# EUROPEAN PATENT APPLICATION

(11) **EP 3 730 477 A1**
(43) Date of publication of application: **28.10.2020**
(21) Application number: 18891594.6
(22) Date of filing: 19.12.2018
(51) Int. Cl.: C07C 309/75, H01L 51/50

(54) **SULFONIC ACID ESTER COMPOUND AND USE OF SAME**

(30) Priority: 20.12.2017 JP 2017243631
(71) Applicant: Nissan Chemical Corporation, Tokyo 103-6119 (JP)
(72) Inventor: OTA, Hirofumi, Funabashi-shi, Chiba 274-0052 (JP); ENDO, Toshiyuki, Funabashi-shi, Chiba 274-0052 (JP)
(74) Representative: Mewburn Ellis LLP
(86) International application number: PCT/JP2018/046687
(87) International publication number: WO 2019/124412

(57) **Abstract**

Provided is a sulfonic acid ester compound which is represented by formula (1). 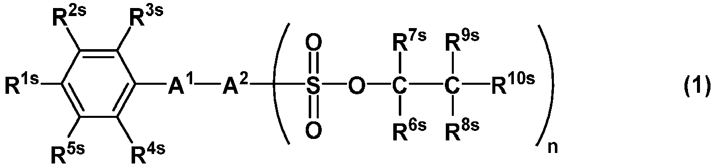 (In the formula, each of R^{1s} -R^{5s} independently represents a hydrogen atom, a nitro group, a cyano group, a halogen atom, an alkyl group, a halogenated alkyl group or a halogenated alkenyl group; each of R^{6s}-R^{9s} independently represents a hydrogen atom or a linear or branched monovalent aliphatic hydrocarbon group; R^{10s} represents a linear or branched monovalent aliphatic hydrocarbon group or -OR^{11s}, wherein R^{11s} represents an optionally substituted monovalent hydrocarbon group having 2-20 carbon atoms; A¹ represents -O-, -S- or -NH-; A² represents an (n + 1)-valent aromatic group; and n represents an integer that satisfies 1 ≤ n ≤ 4.)

## Description

### TECHNICAL FIELD

The present invention relates to a sulfonic acid ester compound and use of the sulfonic acid ester compound.

### BACKGROUND ART

Charge-transporting thin films made of organic compounds are used as light-emitting layers and charge-injecting layers in organic electroluminescent (EL) devices. In particular, a hole-injecting layer is responsible for transferring charge between an anode and a hole-transporting layer or a light-emitting layer, and thus carries out an important function for achieving low-voltage driving and high brightness in organic EL devices.

In the past few years, charge-transporting varnishes composed of a uniform solution of a low-molecular-weight oligoaniline-based material or an oligothiophene-based material dissolved in an organic solvent have been discovered and it has been reported that, by inserting a hole-injecting layer obtained from such a varnish in an organic EL device, an underlying substrate leveling effect and excellent organic EL device properties can be obtained (Patent Documents 1 to 3). Moreover, it has also been reported that, by using a 1,4-benzodioxanesulfonic acid compound as an electron-accepting substance (Patent Documents 4 to 6), the driving voltage of organic EL devices can be lowered.

Yet, because sulfonic acid compounds generally have a low solubility in organic solvents, there tend to be limitations on the solvent used when preparing an organic solution; that is, it has been necessary to include a highly polar organic solvent which has a high solvating power, such as N,N-dimethylacetamide or N-methylpyrrolidone. Organic solutions containing a highly polar organic solvent sometimes cause damage to parts of inkjet coating devices or to organic structures such as insulating films and barrier membranes formed on substrates. Another problem is that, with the prolonged atmospheric exposure of a varnish containing a highly polar organic solvent, the electrical conductivity of the varnish rises over time due to water absorption, as a result of which inkjet discharge becomes unstable. Moreover, because sulfonic acid compounds are highly polar, purification by silica gel column chromatography, liquid/liquid extraction, and salt removal by an operation such as water rinsing are difficult.

At the same time, sulfonic acid ester compounds are known to be materials which have a high solubility in various organic solvents and which generate strong organic acids under external stimulation such as heating or chemical action. The cyclohexyl ester of sulfonic acid has been reported as a specific example of a compound which generates sulfonic acid under heating (Non-Patent Document 1). Notice has also been taken of this sulfonic acid ester compound in terms of the concept of a thermal acid generator (Patent Document 7, Non-Patent Document 2). Yet, particularly with regard to sulfonic acid ester compounds substituted on the electron-deficient aromatic ring of an aromatic disulfonic acid or the like, there has existed a desire for the creation of highly stable sulfonic acid ester compounds that readily decompose under slight heating or via reaction with water, a basic substance or the like.

### PRIOR ART DOCUMENTS

### PATENT DOCUMENTS

Patent Document 1: JP-A 2002-151272
Patent Document 2: WO 2004/043117
Patent Document 3: WO 2005/043962
Patent Document 4: WO 2005/000832
Patent Document 5: WO 2009/096352
Patent Document 6: WO 2015/111654
Patent Document 7: JP-A H07-134416
Patent Document 8: JP No. 5136795

### NON-PATENT DOCUMENTS

Non-Patent Document 1: Chemische Berichte, 90, pp. 585-592 (1957)
Non-Patent Document 2: Kino Zairyo, 24, pp. 72-82 (2004)

### SUMMARY OF INVENTION

### TECHNICAL PROBLEM

The inventors, in order to resolve the above problems, have reported sulfonic acid ester compounds that possess a high stability and also have a high solubility in a wide range of organic solvents (Patent Document 8). However, although these sulfonic acid ester compounds have a better stability and a better solubility in organic solvents than the sulfonic acid compounds and sulfonic acid ester compounds that have hitherto been used, dissolving them in low-polarity solvents requires a high temperature and prolonged stirring. Moreover, when such compounds are formed into a solution, settling occurs with long-term storage. Hence, there has remained room for improvement, both in the solubility of these compounds in organic solvents and in their stability.

It is therefore an object of the invention to provide a sulfonic acid ester compound which has an excellent solubility in low-polarity solvents, which has an excellent stability as a varnish, and which, when employed in organic EL devices, makes it possible to achieve excellent device characteristics. A further object of the invention is to provide an electron-accepting substance precursor consisting of the sulfonic acid ester compound, and a charge-transporting varnish containing such a precursor.

### SOLUTION TO PROBLEM

The inventors have conducted extensive investigations in order to achieve the above object. As a result, they have discovered that esters obtained from specific sulfonic acid compounds and glycol ethers or aliphatic alcohol compounds have an excellent solubility in low-polarity solvents compared with conventional sulfonic acid ester compounds, and moreover, when rendered into solutions, also have an excellent shelf stability, enabling them to function as electron-accepting substance precursors.

Accordingly, the invention provides the following sulfonic acid ester compound, and use of the sulfonic acid ester compound.
1. A sulfonic acid ester compound of the following formula (1): 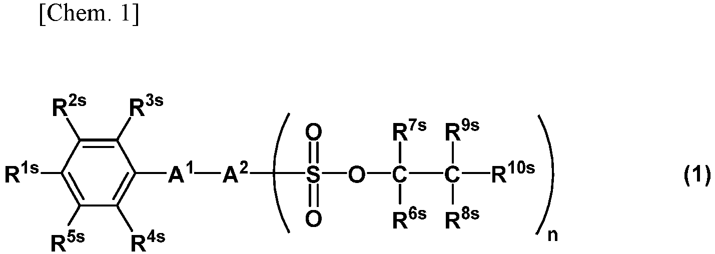
   wherein R^{1s} to R^{5s} are each independently a hydrogen atom, a nitro group, a cyano group, a halogen atom, an alkyl group of 1 to 10 carbon atoms, a halogenated alkyl group of 1 to 10 carbon atoms, or a halogenated alkenyl group of 2 to 10 carbon atoms;
   R^{6s} to R^{9s} are each independently a hydrogen atom, or a linear or branched monovalent aliphatic hydrocarbon group of 1 to 20 carbon atoms;
   R^{10s} is a linear or branched monovalent aliphatic hydrocarbon group of 1 to 20 carbon atoms, or -OR^{11s}, where R^{11s} is an optionally substituted monovalent hydrocarbon group of 2 to 20 carbon atoms;
   A¹ is -O-, -S- or -NH-;
   A² is an (n+1)-valent aromatic group; and
   n is an integer that satisfies the condition 1 ≤ n ≤ 4.
2. The sulfonic acid ester compound of 1 above, wherein R^{1s} is a nitro group, a cyano group, a halogenated alkyl group of 1 to 10 carbon atoms, or a halogenated alkenyl group of 2 to 10 carbon atoms.
3. The sulfonic acid ester compound of 1 or 2 above, wherein all of R^{2s} to R^{5s} are fluorine atoms.
4. The sulfonic acid ester compound of any one of 1 to 3 above, wherein A² is a group derived from naphthalene.
5. The sulfonic acid ester compound of any one of 1 to 4 above, wherein n is 2.
6. The sulfonic acid ester compound of any one of 1 to 5 above, which is represented by the following formula (1-1). 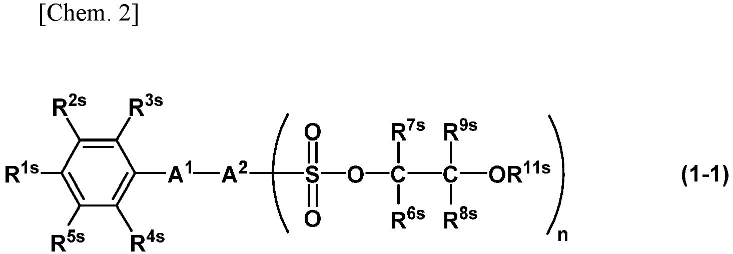 wherein R^{1s} to R^{9s}, R^{11s}, A¹, A² and n are the same as described above.
7. The sulfonic acid ester compound of any one of 1 to 5 above, which is represented by the following formula (1-2). 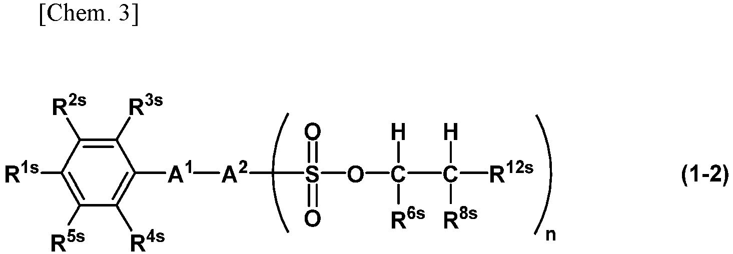 wherein R^{1s} to R^{6s}, R^{8s}, A¹, A² and n are the same as described above, and R^{12s} is a linear or branched monovalent aliphatic hydrocarbon group of 1 to 20 carbon atoms.
8. An electron-accepting substance precursor consisting of the sulfonic acid ester compound of any one of 1 to 7 above.
9. A charge-transporting varnish comprising the electron-accepting substance precursor of 8 above, a charge-transporting substance, and an organic solvent.
10. The charge-transporting varnish of 9 above, wherein the organic solvent is a low-polarity organic solvent.
11. The charge-transporting varnish of 9 or 10 above, wherein the charge-transporting substance is an aniline derivative.
12. A charge-transporting thin film obtained using the charge-transporting varnish of any one of 9 to 11 above.
13. An organic EL device comprising the charge-transporting thin film of 12 above.

### ADVANTAGEOUS EFFECTS OF INVENTION

The sulfonic acid ester compound of the invention has a high solubility in a broad range of organic solvents including low-polarity solvents. Therefore, a charge-transporting varnish can be prepared from this compound even when a low-polarity solvent is used or the proportion of high-polarity solvent is decreased. Moreover, when a solution of the compound is prepared, the shelf stability of the solution is also excellent. Not only can low-polarity organic solvent-based charge-transporting varnishes be applied with inkjet coaters, which have a poor solvent resistance, they can be used even in cases where a structure having a poor solvent resistance, such as an insulating film or a barrier membrane, is present on a substrate. As a result, amorphous solid thin-films having a high flatness can be produced without difficulty. In addition, low-polarity organic solvent-based charge-transporting varnishes lack water absorbing properties and therefore have a long-term atmospheric stability.

Also, because thin films obtained from the charge-transporting varnish of the invention have a high charge transportability, when such a film is used as a hole-injecting layer or a hole-transporting layer, the driving voltage of the organic EL device can be lowered. By taking advantage of the high flatness and high charge transportability of these thin films, it is also possible to employ the thin films as hole-transporting layers in solar cells, as fuel cell electrodes, as protective films for capacitor electrodes, and as antistatic films.

### DESCRIPTION OF EMBODIMENTS

### [Sulfonic Acid Ester Compound]

The sulfonic acid ester compound of the invention is a compound of the following formula (1).

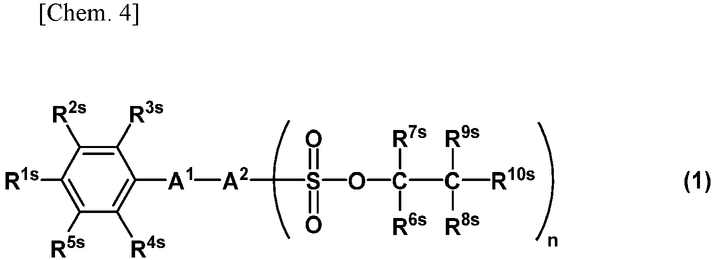

In formula (1), R^{1s} to R^{5s} are each independently a hydrogen atom, a nitro group, a cyano group, a halogen atom, an alkyl group of 1 to 10 carbon atoms, a halogenated alkyl group of 1 to 10 carbon atoms, or a halogenated alkenyl group of 2 to 10 carbon atoms.

The alkyl group of 1 to 10 carbon atoms may be linear, branched or cyclic, and specific examples thereof include a methyl group, an ethyl group, an n-propyl group, an isopropyl group, an n-butyl group, and an isobutyl group, a sec-butyl group, a tert-butyl group, an n-pentyl group, a cyclopentyl group, an n-hexyl group, a cyclohexyl group, an n-heptyl group, an n-octyl group, an n-nonyl group, and an n-decyl group.

The halogenated alkyl group of 1 to 10 carbon atoms is not particularly limited as long as some or all of the hydrogen atoms of the alkyl group of 1 to 10 carbon atoms are substituted with halogen atoms. The halogenated alkyl group may be linear, branched or cyclic, and specific examples thereof include a trifluoromethyl group, a 2,2,2-trifluoroethyl group, a 1,1,2,2,2-pentafluoroethyl group, a 3,3,3-trifluoropropyl group, a 2,2,3,3,3-pentafluoropropyl group, a 1,1,2,2,3,3,3-heptafluoropropyl group, a 4,4,4-trifluorobutyl group, a 3,3,4,4,4-pentafluorobutyl group, a 2,2,3,3,4,4,4-heptafluorobutyl group and a 1,1,2,2,3,3,4,4,4-nonafluorobutyl group.

The halogenated alkenyl group of 2 to 10 carbon atoms is not particularly limited as long as some or all of the hydrogen atoms of the alkenyl group of 2 to 10 carbon atoms are substituted with halogen atoms. Specific examples thereof include a perfluorovinyl group, a perfluoro-1-propenyl group, a perfluoro-2-propenyl group, a perfluoro-1-butenyl group, a perfluoro-2-butenyl group and a perfluoro-3-butenyl group.

Of these, R^{1s} is preferably a nitro group, a cyano group, a halogenated alkyl group of 1 to 10 carbon atoms, a halogenated alkenyl group of 2 to 10 carbon atoms or the like, more preferably a nitro group, a cyano group, a halogenated alkyl group of 1 to 4 carbon atoms, a halogenated alkenyl group of 2 to 4 carbon atoms, or the like, still more a nitro group, a cyano group, a trifluoromethyl group, a perfluoro-1-propenyl group, a perfluoro-2-propenyl group or the like. R^{2s} to R^{5s} are each preferably a halogen atom, more preferably a fluorine atom.

In formula (1), R^{6s} to R^{9s} are each independently a hydrogen atom, or a linear or branched monovalent aliphatic hydrocarbon group of 1 to 20 carbon atoms.

Examples of the monovalent aliphatic hydrocarbon group include alkyl groups of 1 to 20 carbon atoms such as a methyl group, an ethyl group, an n-propyl group, an isopropyl group, an n-butyl group, and an isobutyl group, a sec-butyl group, a tert-butyl group, an n-pentyl group, an n-hexyl group, an n-heptyl group, an n-octyl group, an n-nonyl group, an n-decyl group, an n-undecyl group and an n-dodecyl group; and alkenyl groups of 2 to 20 carbon atoms such as a vinyl group, a 1-propenyl group, a 2-propenyl group, an isopropenyl group, a 1-methyl-2-propenyl group, a 1-butenyl group, a 2-butenyl group, a 3-butenyl group and a hexenyl group. Of these, alkyl groups of 1 to 20 carbon atoms are preferable, alkyl groups of 1 to 10 carbon atoms are more preferable, and alkyl groups of 1 to 8 carbon atoms are still more preferable.

In formula (1), R^{10s} is a linear or branched monovalent aliphatic hydrocarbon group of 1 to 20 carbon atoms, or -OR^{11s}. R^{11s} is an optionally substituted monovalent hydrocarbon group of 2 to 20 carbon atoms.

Examples of the linear or branched monovalent aliphatic hydrocarbon group of 1 to 20 carbon atoms, which is represented by R^{10s}, include the same as those mentioned above. When R^{10s} is a monovalent aliphatic hydrocarbon group, R^{10s} is preferably an alkyl group of 1 to 20 carbon atoms, more preferably an alkyl group of 1 to 10 carbon atoms, still more preferably an alkyl group of 1 to 8 carbon atoms.

Examples of the monovalent hydrocarbon group of 2 to 20 carbon atoms, which is represented by R^{11s}, include the above-mentioned monovalent aliphatic hydrocarbon groups except for a methyl group, and aryl groups such as a phenyl group, a naphthyl group and a phenanthryl group. Of these, R^{11s} is preferably a linear alkyl group of 2 to 4 carbon atoms or a phenyl group. Examples of the substituent optionally present in the monovalent hydrocarbon group include a fluoro group, an alkoxy group of 1 to 4 carbon atoms, a nitro group and a cyano group.

In the formula (1), A¹ is -O-, -S- or -NH-, preferably -O-.

In the formula (1), A² is an (n+1)-valent aromatic group. The aromatic group is a group obtained by removing n+1 hydrogen atoms from an aromatic ring of an aromatic compound. Examples of the aromatic compound include benzene, toluene, xylene, naphthalene, anthracene and phenanthrene. Of these, A² is preferably a group derived from naphthalene or anthracene, more preferably a group derived from naphthalene.

In formula (1), n is an integer which satisfies the condition 1 ≤ n ≤ 4, and n is preferably 2.

The sulfonic acid ester compound of formula (1) is particularly preferably a compound of the following formula (1-1) or (1-2).

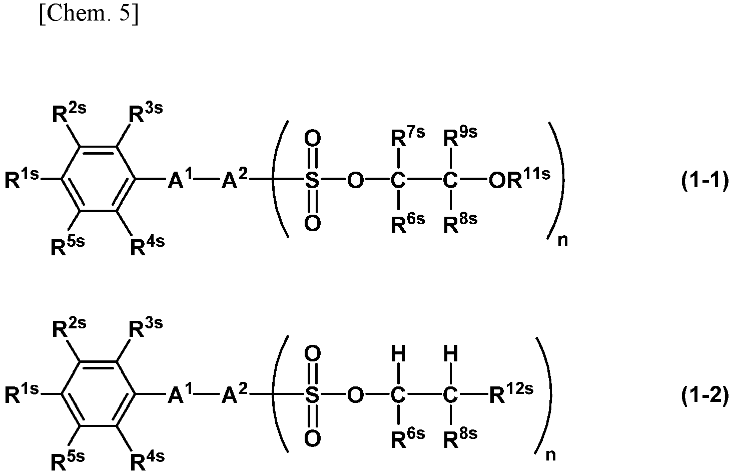

In the formula, R^{1s} to R^{9s}, R^{11s}, A¹, A² and n are the same as described above. R^{12s} is a linear or branched monovalent aliphatic hydrocarbon group of 1 to 20 carbon atoms, and specific examples thereof include the same as those mentioned above for R^{10s}.

In the sulfonic acid ester compound of formula (1-1), it is preferable that among R^{6s} to R^{9s}, R^{6s} or R^{8s} is a linear alkyl group of 1 to 3 carbon atoms, and the remainder is a hydrogen atom. Further, it is preferable that R^{6s} is a linear alkyl group of 1 to 3 carbon atoms, and R^{7s} to R^{9s} are hydrogen atoms. The linear alkyl group of 1 to 3 carbon atoms is preferably a methyl group. R^{11s} is preferably a linear alkyl group of 2 to 4 carbon atoms or a phenyl group.

In the sulfonic acid ester compound of formula (1-2), the total number of carbon atoms of R^{6s}, R^{8s} and R^{12s} is preferably 6 or more. The upper limit of the total number of carbon atoms of R^{6s}, R^{8s} and R^{12s} is preferably 20 or less, and more preferably 10 or less. Here, R^{6s} is preferably a hydrogen atom, and R^{8s} to R^{12s} are each preferably an alkyl group of 1 to 6 carbon atoms. R^{8s} and R^{12s} may be identical to or different from each other.

Because the sulfonic acid ester compound of formula (1) exhibits a high solubility in a broad range of solvents including low-polarity solvents, the physical properties of the solution can be adjusted using a variety of solvents, and the solution has a high coatability. Therefore, it is preferable for application to be carried out while the solution is in the state of a sulfonic acid ester, and for sulfonic acid to be generated when the applied film is dried or fired. Because it is desirable for the sulfonic acid ester to be stable at room temperature and at or below the firing temperature, the temperature at which sulfonic acid is generated from the sulfonic acid ester is typically from 40 to 260°C. Taking into account the high stability of the sulfonic acid ester within the varnish and the ease of dissociation during firing, the temperature is preferably from 80 to 230°C, and more preferably from 120 to 180°C.

The sulfonic acid ester compound of formula (1) can be synthesized by, for example, as shown in Scheme A below, reacting a sulfonic acid salt compound of formula (1") with a halogenating agent so as to synthesize a sulfonyl halide compound of formula (1') below (referred to below as "Step 1"), and then reacting this sulfonyl halide compound with a compound of formula (2) (referred to below as "Step 2"). 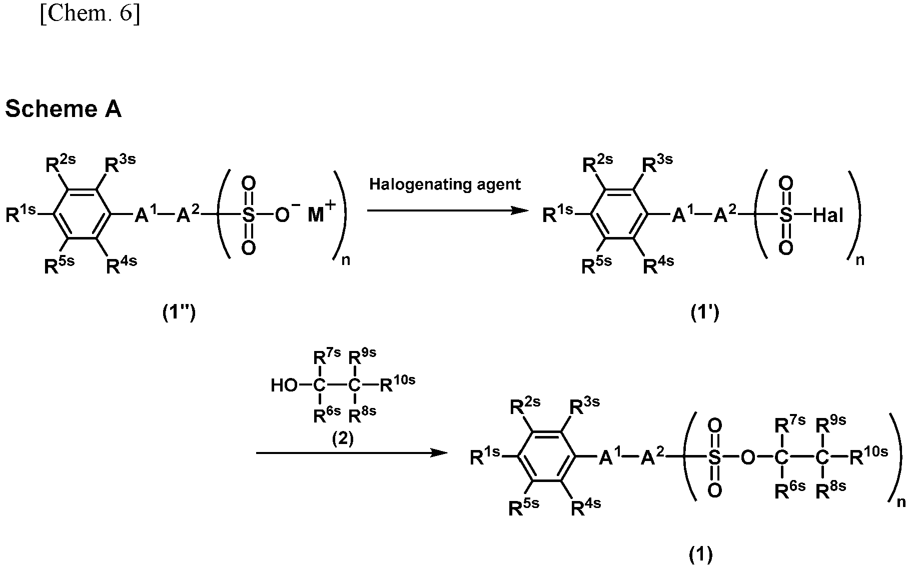 Herein, R^{1s} to R^{10s}, A¹, A² and n are the same as described above; M⁺ is a monovalent cation such as a sodium ion, a potassium ion, a pyridinium ion or a quaternary ammonium ion; and Hal is a halogen atom such as a chlorine atom and a bromine atom.

The sulfonic acid salt compound of formula (1") can be synthesized by a known method.

Examples of the halogenating agent used in Step 1 include thionyl chloride, oxalyl chloride, phosphorus oxychloride and phosphorus(V) chloride; thionyl chloride is preferred. The amount of halogenating agent used is not limited, so long as it is at least one mole per mole of the sulfonic acid salt compound, although use in an amount that, expressed as a weight ratio, is from 2 to 10 times the amount of the sulfonic acid salt compound is preferred.

The reaction solvent used in Step 1 is preferably a solvent that does not react with the halogenating agent, examples of which include chloroform, dichloroethane, carbon tetrachloride, hexane and heptane. The reaction can be carried out without a solvent, and here, the halogenating agent is preferably used in at least the amount at which the system becomes a uniform solution at the time of reaction completion. Further, a catalyst such as N,N-dimethylformamide may be used for accelerating the reaction. The reaction temperature may be set to from about 0°C to about 150°C, although the reaction temperature is preferably from 20 to 100°C and at or below the boiling point of the halogenating agent used. Following reaction completion, the crude product obtained by vacuum concentration or the like is generally used in the next step.

Among the compounds of formula (2), those in which R^{10s} is -OR^{11s} include glycol ethers such as propylene glycol monoethyl ether, propylene glycol monopropyl ether, propylene glycol monobutyl ether, propylene glycol monophenyl ether, ethylene glycol monobutyl ether and ethylene glycol monohexyl ether. Among the compounds of formula (2), those in which R^{10s} is a linear or branched monovalent hydrocarbon group of 1 to 20 carbon atoms include alcohols such as 2-ethyl-1-hexanol, 2-butyl-1-octanol, 1-octanol and 3-nonanol.

In Step 2, a base may be concomitantly used. Examples of bases that may be used include sodium hydride, pyridine, triethylamine and diisopropylethylamine. Sodium hydride, pyridine and triethylamine are preferred. The base is preferably used in an amount that ranges from one mole per mole of the sulfonyl halide compound (1') up to the amount of solvent.

Various organic solvents may be used as the reaction solvent in Step 2, although tetrahydrofuran, dichloroethane, chloroform and pyridine are preferred. The reaction temperature, although not particularly limited, is preferably from 0 to 80°C. Following reaction completion, pure sulfonic acid ester compound can be obtained by work-up and purification using customary methods such as vacuum concentration, liquid/liquid extraction, water rinsing, reprecipitation, recrystallization and chromatography. The pure sulfonic acid ester compound thus obtained can be rendered into a high-purity sulfonic acid compound by being subjected to heat treatment or the like.

Alternatively, as shown in Scheme B below, the sulfonic acid ester compound of formula (1) can be synthesized from a sulfonic acid compound of formula (1'''). In the Scheme B below, the halogenating agent, compound of formula (2), reaction solvent and other ingredients used in the first-stage and second-stage reactions may be the same as those used in Steps 1 and 2 of Reaction Scheme A. 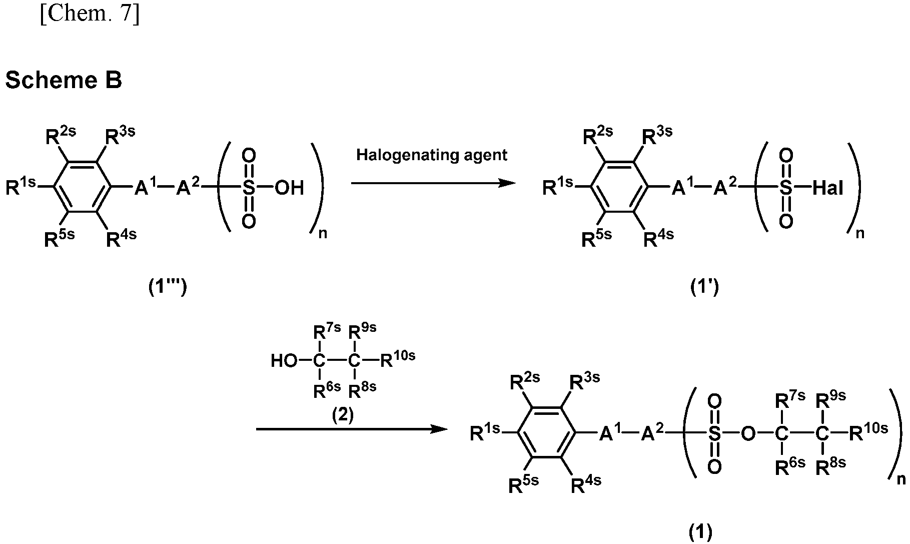 Herein, R^{1s} to R^{10s}, A¹, A², n and Hal are the same as described above.

The sulfonic acid compound of formula (1''') may be synthesized according to, for example, the method described in WO 2006/025342.

### [Electron-Accepting Substance Precursor]

The sulfonic acid ester compound of formula (1) is suitably used as an acid generator or an electron-accepting substance precursor because sulfonic acid is generated by heat treatment or the like and the sulfonic acid compound has an electron-accepting property. Here, the electron-accepting substance is used for enhancing the electron transporting ability and increasing the uniformity of film formation, and is synonymous with an electron-accepting dopant.

Because the sulfonic acid ester compound of formula (1) exhibits a high solubility in a broad range of solvents, including low-polarity solvents, the physical properties of the solution can be adjusted using a variety of solvents, and the solution has a high coatability. Therefore, it is preferable for application to be carried out while the solution is in the state of a sulfonic acid ester, and for sulfonic acid to be generated when the applied film is dried or fired. Because it is desirable for the sulfonic acid ester to be stable at room temperature and at or below the firing temperature, the temperature at which sulfonic acid is generated from the sulfonic acid ester is typically from 40 to 260°C. Taking into account the high stability of the sulfonic acid ester within the varnish and the ease of dissociation during firing, the temperature is preferably from 80 to 230°C, and more preferably from 120 to 180°C.

The sulfonic acid ester compound of formula (1) can be rendered into a charge-transporting varnish by dissolution or dispersion, together with the charge-transporting substance serving as the central part of the charge transport mechanism, in an organic solvent. The sulfonic acid ester compounds may be used singly, or in combination of two or more thereof.

### [Charge-Transporting Varnish]

The charge-transporting varnish of the invention includes an electron-accepting substance precursor consisting of the compound of formula (1), a charge-transporting substance, and an organic solvent. In this invention, "charge-transportability" is synonymous with electrical conductivity. Also, "charge-transporting varnish" may refer to a varnish which itself has charge transportability or to one from which there can be obtained a solid film having charge transportability.

### [Charge-Transporting Substance]

A charge-transporting substance hitherto used in the organic EL field may be used as the above charge-transporting substance. Specific examples thereof include charge-transporting oligomers such as aniline derivatives, thiophene derivatives and pyrrole derivatives. The molecular weight of the charge-transporting oligomer is typically from 200 to 8,000. From the standpoint of preparing a varnish which gives thin films having a high charge transportability, the molecular weight is preferably at least 300, more preferably at least 400, and even more preferably at least 500. From the standpoint of preparing a uniform varnish that gives thin films having a high flatness, the molecular weight is preferably not more than 6,000, more preferably not more than 5,000, even more preferably not more than 4,000, and still more preferably not more than 3,000.

Among the above charge-transporting oligomers, aniline derivatives are preferred in view of the balance between the solubility in organic solvents and the charge transportability of the resulting thin film. Exemplary aniline derivatives include the oligoaniline derivatives mentioned in JP-A 2002-151272, the oligoaniline compounds mentioned in WO 2004/105446, the oligoaniline compounds mentioned in WO 2008/032617, the oligoaniline compounds mentioned in WO 2008/032616, the aryldiamine compounds mentioned in WO 2013/042623, and the aniline derivatives mentioned in WO 2015/050253 and WO 2016/190326.

The aniline derivative used may be, for example, a compound of formula (A1) or (A2).

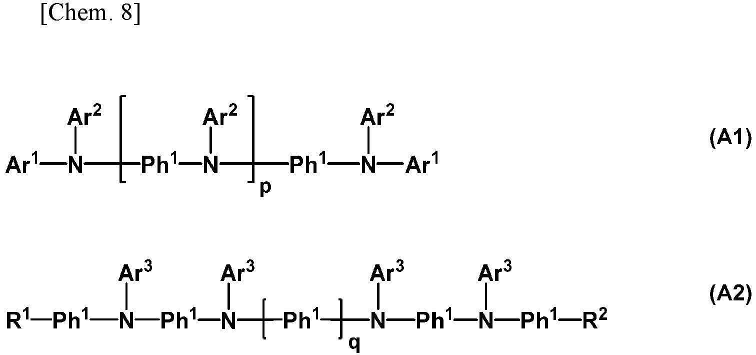

In the formula (A2), R¹ and R² are each independently a hydrogen atom, a halogen atom, a nitro group, a cyano group, or an alkyl group of 1 to 20 carbon atoms, alkenyl group of 2 to 20 carbon atoms, alkynyl group of 2 to 20 carbon atoms, aryl group of 6 to 20 carbon atoms or heteroaryl group of 2 to 20 carbon atoms which is optionally substituted with a halogen atom.

Examples of the halogen atom include fluorine, chlorine, bromine and iodine atoms.

The alkyl group of 1 to 20 carbon atoms may be linear, branched or cyclic. Specific examples thereof include linear or branched alkyl groups of 1 to 20 carbon atoms such as methyl, ethyl, n-propyl, isopropyl, n-butyl, isobutyl, sec-butyl, tert-butyl, n-pentyl, n-hexyl, n-heptyl, n-octyl, n-nonyl and n-decyl groups; and cyclic alkyl groups of 3 to 20 carbon atoms such as cyclopropyl, cyclobutyl, cyclopentyl, cyclohexyl, cycloheptyl, cyclooctyl, cyclononyl, cyclodecyl, bicyclobutyl, bicyclopentyl, bicyclohexyl, bicycloheptyl, bicyclooctyl, bicyclononyl and bicyclodecyl groups.

The alkenyl group of 2 to 20 carbon atoms may be linear, branched or cyclic. Specific examples thereof include vinyl, n-1-propenyl, n-2-propenyl, 1-methylvinyl, n-1-butenyl, n-2-butenyl, n-3-butenyl, 2-methyl-1-propenyl, 2-methyl-2-propenyl, 1-ethylvinyl, 1 methyl-1-propenyl, 1-methyl-2-propenyl, n-1-pentenyl, n-1-decenyl and n-1-eicosenyl groups.

The alkynyl group of 2 to 20 carbon atoms may be linear, branched or cyclic. Specific examples thereof include ethynyl, n-1-propynyl, n-2-propynyl, n-1-butynyl, n-2-butynyl, n-3-butynyl, 1-methyl-2-propynyl, n-1-pentynyl, n-2-pentynyl, n-3-pentynyl, n-4-pentynyl, 1-methyl-n-butynyl, 2-methyl-n-butynyl, 3-methyl-n-butynyl, 1,1-dimethyl-n-propynyl, n 1-hexynyl, n-1-decynyl, n-1-pentadecynyl and n-1-eicosynyl groups.

Specific examples of the aryl group of 6 to 20 carbon atoms include phenyl, 1-naphthyl, 2-naphthyl, 1-anthryl, 2-anthryl, 9-anthryl, 1-phenanthryl, 2-phenanthryl, 3-phenanthryl, 4-phenanthryl and 9-phenanthryl groups.

Specific examples of the heteroaryl group of 2 to 20 carbon atoms include 2-thienyl, 3-thienyl, 2-furanyl, 3-furanyl, 2-oxazolyl, 4-oxazolyl, 5-oxazolyl, 3-isooxazolyl, 4-isooxazolyl, 5-isooxazolyl, 2-thiazolyl, 4-thiazolyl, 5-thiazolyl, 3-isothiazolyl, 4-isothiazolyl, 5-isothiazolyl, 2-imidazolyl, 4-imidazolyl, 2-pyridyl, 3-pyridyl and 4-pyridyl groups.

Of these, R¹ and R² are preferably hydrogen atoms, fluorine atoms, cyano groups, alkyl groups of 1 to 20 carbon atoms which are optionally substituted with halogen atoms, aryl groups of 6 to 20 carbon atoms which are optionally substituted with halogen atoms, or heteroaryl groups of 2 to 20 carbon atoms which are optionally substituted with halogen atoms; more preferably hydrogen atoms, fluorine atoms, cyano groups, alkyl groups of 1 to 10 carbon atoms which are optionally substituted with halogen atoms, or phenyl groups which are optionally substituted with halogen atoms; even more preferably hydrogen atoms or fluorine atoms; and most preferably hydrogen atoms.

In the formulas (A1) and (A2), Ph¹ is a group of formula (PI).

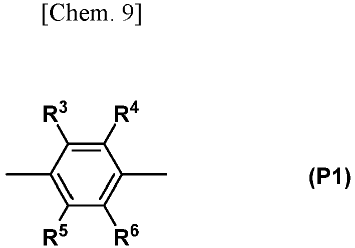

In the formula, R³ and R⁶ are each independently a hydrogen atom, a halogen atom, a nitro group, a cyano group, or an alkyl group of 1 to 20 carbon atoms, alkenyl group of 2 to 20 carbon atoms, alkynyl group of 2 to 20 carbon atoms, aryl group of 6 to 20 carbon atoms or heteroaryl group of 2 to 20 carbon atoms which is optionally substituted with a halogen atom. Specific examples thereof include the same as those mentioned above for R¹ and R².

In particular, R³ to R⁶ are preferably hydrogen atoms, fluorine atoms, cyano groups, alkyl groups of 1 to 20 carbon atoms which are optionally substituted with halogen atoms, aryl groups of 6 to 20 carbon atoms which are optionally substituted with halogen atoms, or heteroaryl groups of 2 to 20 carbon atoms which are optionally substituted with halogen atoms; more preferably hydrogen atoms, fluorine atoms, cyano groups, alkyl groups of 1 to 10 carbon atoms which are optionally substituted with halogen atoms, or phenyl groups which are optionally substituted with halogen atoms; even more preferably hydrogen atoms or fluorine atoms; and most preferably hydrogen atoms.

Examples of suitable groups for Ph¹ include, but are not limited to, a 1,4-phenylene group.

Each Ar¹ in formula (A1) is independently a group of any of formulas (B1) to (B 11), and more preferably a group of any of formulas (B1') to (B11').

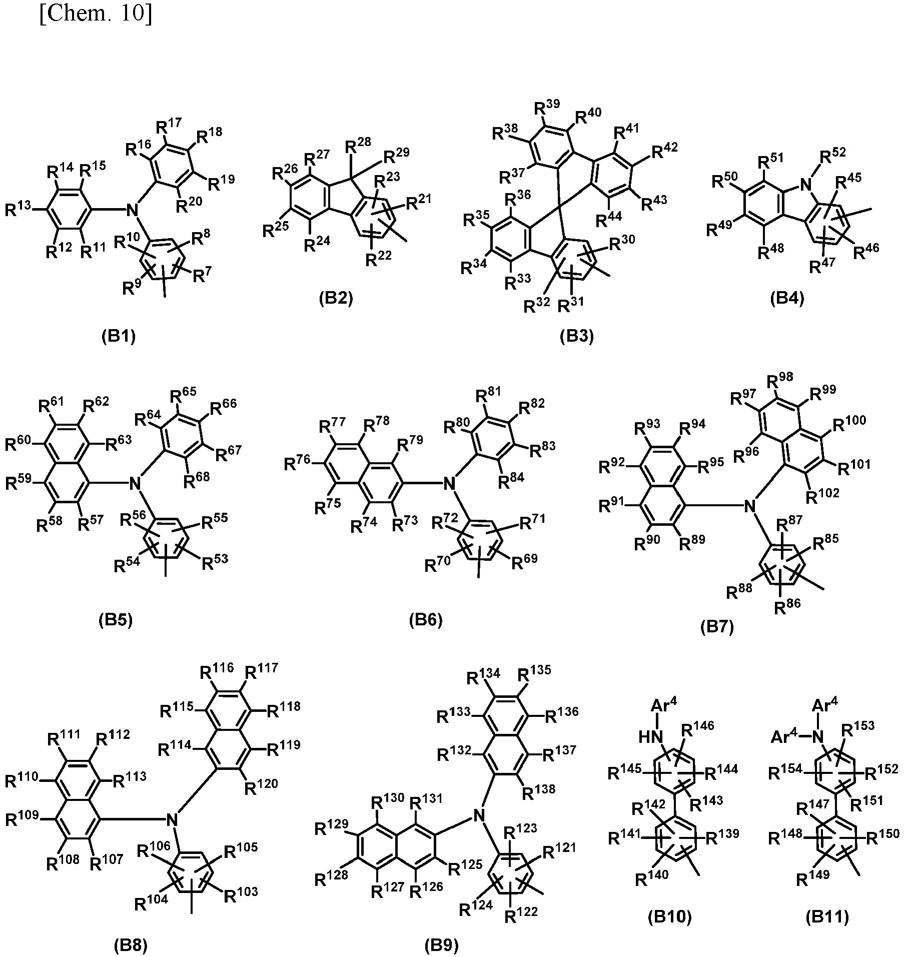

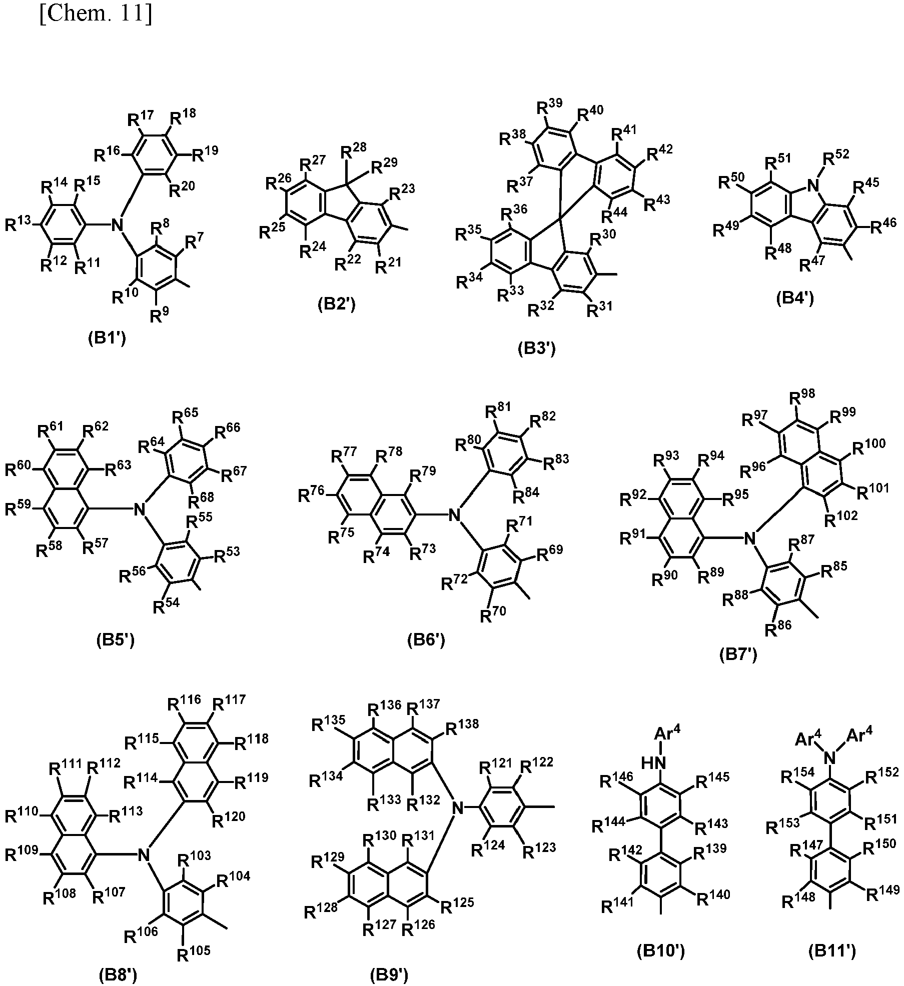

In formulas (B1) to (B11) and (B1') to (B11'), R⁷ to R²⁷, R³⁰ to R⁵¹ and R⁵³ to R¹⁵⁴ are each independently a hydrogen atom, a halogen atom, a nitro group, a cyano group, or a diphenylamino group, an alkyl group of 1 to 20 carbon atoms, alkenyl group of 2 to 20 carbon atoms, alkynyl group of 2 to 20 carbon atoms, aryl group of 6 to 20 carbon atoms or heteroaryl group of 2 to 20 carbon atoms which is optionally substituted with a halogen atom. R²⁸ and R²⁹ are each independently an aryl group of 6 to 20 carbon atoms or a heteroaryl group of 2 to 20 carbon atoms which is optionally substituted with Z¹. R⁵² is an aryl group of 6 to 20 carbon atoms or a heteroaryl group of 2 to 20 carbon atoms which is optionally substituted with Z¹.

Z¹ is a halogen atom, a nitro group or a cyano group, or an alkyl group of 1 to 20 carbon atoms, an alkenyl group of 2 to 20 carbon atoms, or an alkynyl group of 2 to 20 carbon atoms which is optionally substituted with Z². Z² is a halogen atom, a nitro group or a cyano group, or an aryl group of 6 to 20 carbon atoms or a heteroaryl group of 2 to 20 carbon atoms which is substituted with Z³. Z³ is a halogen atom, a nitro group or a cyano group.

In particular, R⁷ to R²⁷, R³⁰ to R⁵¹ and R⁵³ to R¹⁵⁴ are preferably hydrogen atoms, fluorine atoms, cyano groups, diphenylamino groups optionally substituted with halogen atoms, alkyl groups of 1 to 20 carbon atoms which are optionally substituted with halogen atoms, aryl groups of 6 to 20 carbon atoms which are optionally substituted with halogen atoms, or heteroaryl groups of 2 to 20 carbon atoms which are optionally substituted with halogen atoms; more preferably hydrogen atoms, fluorine atoms, cyano groups, alkyl groups of 1 to 10 carbon atoms which are optionally substituted with halogen atoms, or phenyl groups which are optionally substituted with halogen atoms; even more preferably hydrogen atoms or fluorine atoms; and most preferably hydrogen atoms.

R²⁸ and R²⁹ are preferably aryl groups of 6 to 14 carbon atoms which are optionally substituted with halogen atoms or heteroaryl groups of 2 to 14 carbon atoms which are optionally substituted with halogen atoms; more preferably phenyl groups optionally substituted with halogen atoms or naphthyl groups optionally substituted with halogen atoms; even more preferably phenyl groups optionally substituted with halogen atoms; and still more preferably phenyl groups.

R⁵² is preferably a hydrogen atom or an aryl group of 6 to 20 carbon atoms which is optionally substituted with Z¹; more preferably a hydrogen atom, a phenyl group optionally substituted with Z¹, or a naphthyl group optionally substituted with Z¹; even more preferably a phenyl group optionally substituted with Z¹; and still more preferably a phenyl group.

Each Ar⁴ in formulae (B10), (B11), (B10') and (B11') is independently an aryl group of 6 to 20 carbon atoms, which is optionally substituted with a diarylamino group in which each aryl group is an aryl group of 6 to 20 carbon atoms. Specific examples of the aryl group of 6 to 20 carbon atoms include the same as those mentioned above for R¹ and R². Specific examples of the diarylamino group include diphenylamino, 1-naphthylphenylamino, di(1-naphthyl)amino, 1-naphthyl-2-naphthylamino and di(2-naphthyl)amino groups.

Ar⁴ is preferably a phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, p-(diphenylamino)phenyl, p-(1-naphthylphenylamino)phenyl, p-(di(1-naphthyl)amino)phenyl, p-(1-naphthyl-2-naphthylamino)phenyl or p-(di(2-naphthyl)amino)phenyl group; and more preferably a p-(diphenylamino)phenyl group.

Each Ar² in formula (A1) is independently a group of any of formulas (C1) to (C18), and particularly preferably a group of any of formulas (C1'-1) to (C18'-2). In the following formula, Ar⁴ is the same as described above, and DPA is a diphenylamino group.

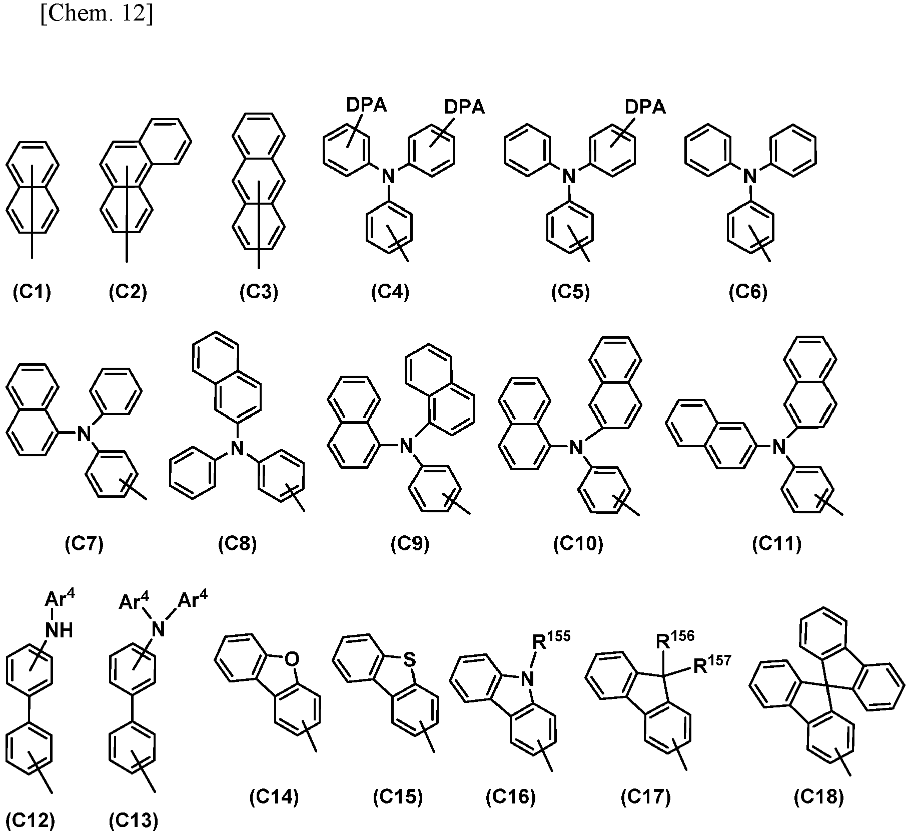

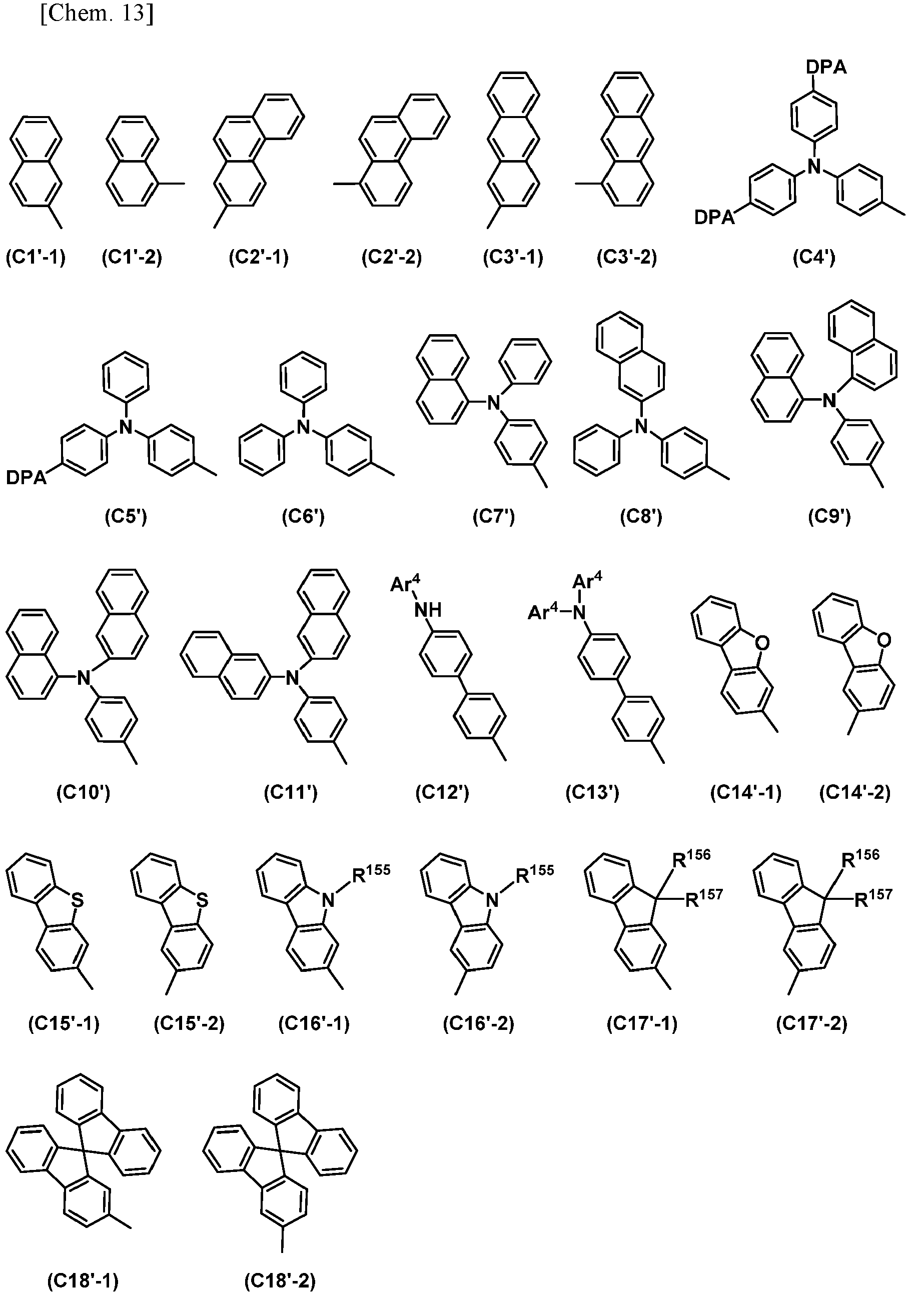

In the formulae (C16), (C16'-1) and (C16'-2), R¹⁵⁵ is a hydrogen atom, an aryl group of 6 to 14 carbon atoms which is optionally substituted with Z¹, or a heteroaryl group of 2 to 14 carbon atoms which is optionally substituted with Z¹. Examples of the aryl group and the heteroaryl group include the same as those mentioned above. Of these, R¹⁵⁵ is preferably a hydrogen atom, a phenyl group optionally substituted with Z¹, a 1-naphthyl group optionally substituted with Z¹, a 2-naphthyl group optionally substituted with Z¹, a 2-pyridyl group optionally substituted with Z¹, a 3-pyridyl group optionally substituted with a phenyl group optionally substituted with Z¹, or a 4-pyridyl group optionally substituted with Z¹; even more preferably a phenyl group optionally substituted with Z¹; and even more preferably a phenyl group or a (2,3,5,6-tetrafluoro-4-(trifluoromethyl)phenyl) group.

In formulae (C17), (C17'-1) and (C17'-2), R¹⁵⁶ and R¹⁵⁷ are aryl groups of 6 to 14 carbon atoms which are optionally substituted with phenyl groups optionally substituted with Z¹, or heteroaryl groups of 2 to 14 carbon atoms which are optionally substituted with phenyl groups optionally substituted with Z¹. Examples of the aryl group and the heteroaryl group include the same as those mentioned above. Of these, R¹⁵⁶ and R¹⁵⁷ are preferably aryl groups of 6 to 14 carbon atoms which are optionally substituted with phenyl groups optionally substituted with Z¹; and more preferably phenyl groups optionally substituted with phenyl groups optionally substituted with Z¹, 1-naphthyl groups optionally substituted with phenyl groups optionally substituted with Z¹, or 2-naphthyl groups optionally substituted with Z¹.

In the formula (A2), Ar³ is a group of any of formulae (D1) to (D8), and particularly preferably a group of any of (D1') to (D8').

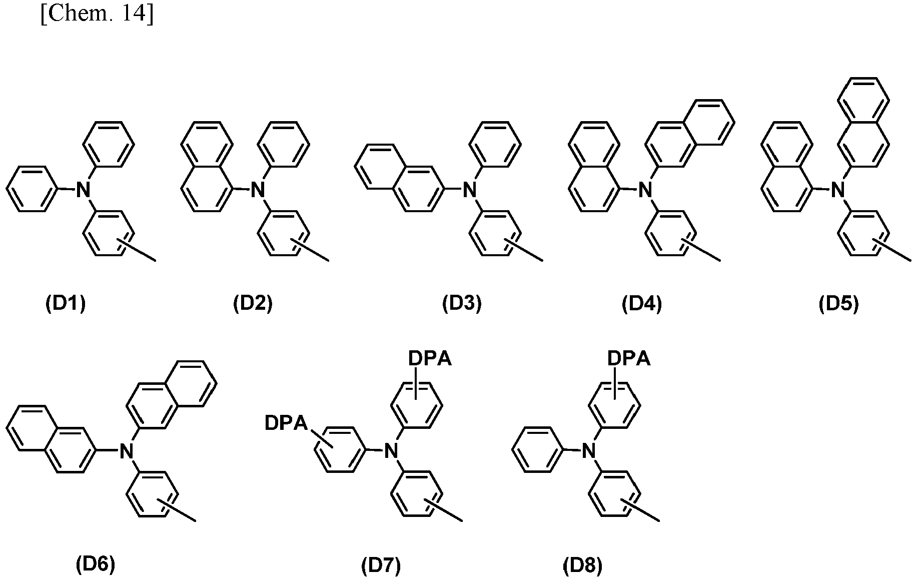

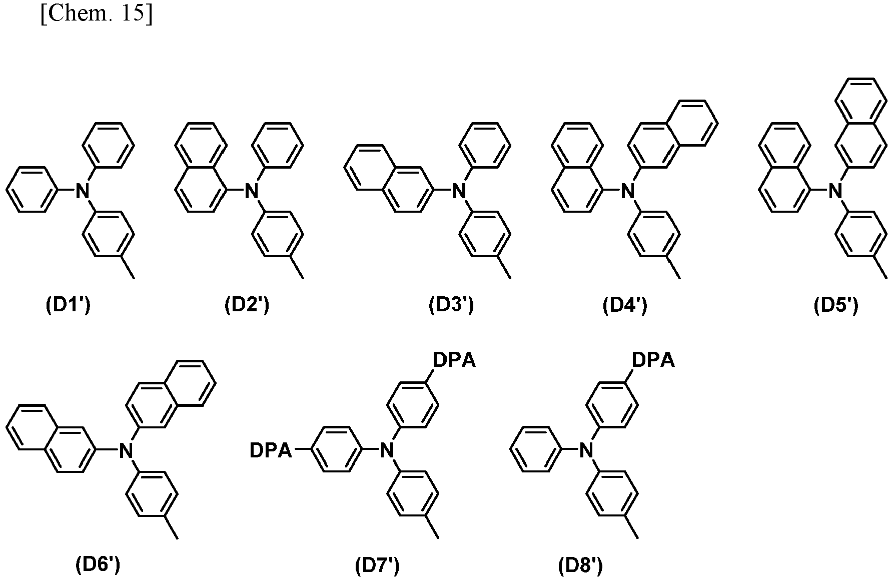

In formula (A1), the subscript is an integer from 1 to 10. From the standpoint of increasing the solubility of the compound in organic solvents, p is preferably from 1 to 5, more preferably from 1 to 3, even more preferably 1 or 2, and most preferably 1. In formula (A2), q is 1 or 2.

The aniline derivative of formula (A1) and the aniline derivative of formula (A2) can be produced according to, for example, the method described in WO 2015/050253.

As the aniline derivatives, fluorine atom-containing oligoaniline derivatives of formula (A3) below can be used.

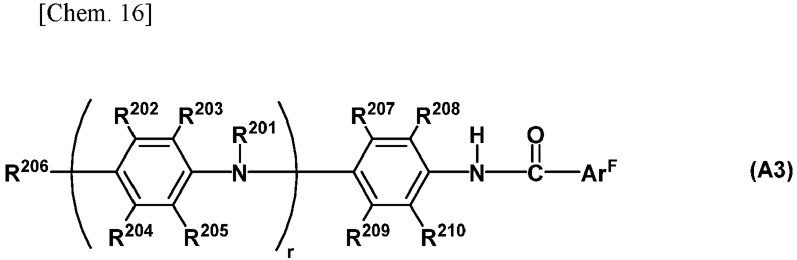

In the formula R²⁰¹ is a hydrogen atom, or an alkyl group of 1 to 20 carbon atoms which is optionally substituted with Z¹¹. Z¹¹ is a halogen atom, a nitro group, a cyano group, an aldehyde group, a hydroxyl group, a thiol group, a sulfonic acid group, a carboxyl group, an aryl group of 6 to 20 carbon atoms which is optionally substituted with Z¹² or a heteroaryl group of 2 to 20 carbon atoms which is optionally substituted with Z¹². Z¹² is a halogen atom, a nitro group, a cyano group, an aldehyde group, a hydroxyl group, a thiol group, a sulfonic acid group or a carboxyl group.

R²⁰² and R²¹⁰ are each independently a hydrogen atom, a halogen atom, a nitro group, a cyano group, or an alkyl group of 1 to 20 carbon atoms, alkenyl group of 2 to 20 carbon atoms, alkynyl group of 2 to 20 carbon atoms, aryl group of 6 to 20 carbon atoms or heteroaryl group of 2 to 20 carbon atoms which is optionally substituted with a halogen atom.

Examples of the halogen atom, alkyl group of 1 to 20 carbon atoms, alkenyl group of 2 to 20 carbon atoms, alkynyl group of 2 to 20 carbon atoms, aryl group of 6 to 20 carbon atoms and heteroaryl group of 2 to 20 carbon atoms include the same as those mentioned above.

Of these, taking into account the solubility of the fluorine atom-containing oligoaniline derivative in organic solvents, R²⁰¹ is preferably a hydrogen atom or an alkyl group of 1 to 10 carbon atoms which is optionally substituted with Z¹¹; more preferably a hydrogen atom or an alkyl group of 1 to 4 carbon atoms which is substituted with Z¹¹; and most preferably a hydrogen atom. When R²⁰¹ is a hydrogen atom, particularly excellent charge transportability can be achieved. In cases where there are a plurality of R²⁰¹ moieties, they may each be the same or may be different.

Of these, taking into account the solubility of the fluorine atom-containing oligoaniline derivative in organic solvents, R²⁰² to R²¹⁰ are preferably hydrogen atoms, halogen atoms, nitro groups, cyano groups or alkyl groups of 1 to 10 carbon atoms which are optionally substituted with halogen atoms; and more preferably hydrogen atoms, halogen atoms or alkyl groups of 1 to 4 carbon atoms which are optionally substituted with halogen atoms. Taking into account the balance between the solubility of the oligoaniline derivative in organic solvents and the charge transportability, R²⁰² to R²¹⁰ are most preferably hydrogen atoms. In cases where there are a plurality of R²⁰² to R²¹⁰ moieties, they may each be the same or may be different. In cases where there are a plurality of R²⁰² to R²⁰⁵ moieties, they may each be the same or may be different.

In formula (A3), Ar^{F} is a fluoroalkyl group of 1 to 20 carbon atoms, fluorocycloalkyl group of 3 to 20 carbon atoms, fluorobicycloalkyl group of 4 to 20 carbon atoms, fluoroalkenyl group of 2 to 20 carbon atoms or fluoroalkynyl group of 2 to 20 carbon atoms which is optionally substituted with a cyano group, chlorine atom, bromine atom, iodine atom, nitro group or fluoroalkoxy group of 1 to 20 carbon atoms; a fluoroaryl group of 6 to 20 carbon atoms which is substituted with a cyano group, chlorine atom, bromine atom, iodine atom, nitro group, alkyl group of 1 to 20 carbon atoms, fluoroalkyl group of 1 to 20 carbon atoms or fluoroalkoxy group of 1 to 20 carbon atoms;
an aryl group of 6 to 20 carbon atoms which is optionally substituted with a fluoroalkyl group of 1 to 20 carbon atoms, fluorocycloalkyl group of 3 to 20 carbon atoms, fluorobicycloalkyl group of 4 to 20 carbon atoms, fluoroalkenyl group of 2 to 20 carbon atoms or fluoroalkynyl group of 2 to 20 carbon atoms and substituted with a cyano group, halogen atom or fluoroalkoxy group of 1 to 20 carbon atoms (hereinafter also referred to a substituted aryl group for the sake of convenience);
a fluoroaralkyl group of 7 to 20 carbon atoms which is optionally substituted with a cyano group, chlorine atom, bromine atom, iodine atom, nitro group, fluoroalkoxy group of 1 to 20 carbon atoms, fluoroalkyl group of 1 to 20 carbon atoms, fluorocycloalkyl group of 3 to 20 carbon atoms, fluorobicycloalkyl group of 4 to 20 carbon atoms, fluoroalkenyl group of 2 to 20 carbon atoms or fluoroalkynyl group of 2 to 20 carbon atoms; or
an aralkyl group of 7 to 20 carbon atoms which is optionally substituted with a fluoroalkyl group of 1 to 20 carbon atoms, fluorocycloalkyl group of 3 to 20 carbon atoms, fluorobicycloalkyl group of 4 to 20 carbon atoms, fluoroalkenyl group of 2 to 20 carbon atoms or fluoroalkynyl group of 2 to 20 carbon atoms and substituted with a cyano group, a halogen atom or a fluoroalkoxy group of 1 to 20 carbon atoms (hereinafter also referred to a substituted aralkyl group for the sake of convenience).

The fluoroalkyl group is not particularly limited, provided that it is a linear or branched alkyl group in which at least one hydrogen atom on a carbon atom is substituted with a fluorine atom. Examples thereof include fluoromethyl, difluoromethyl, trifluoromethyl, 1-fluoroethyl, 2-fluoroethyl, 1,2-difluoroethyl, 1,1-difluoroethyl, 2,2-difluoroethyl, 1,1,2-trifluoroethyl, 1,2,2-trifluoroethyl, 2,2,2-trifluoroethyl, 1,1,2,2-tetrafluoroethyl, 1,2,2,2-tetrafluoroethyl, 1,1,2,2,2-pentafluoroethyl, 1-fluoropropyl, 2-fluoropropyl, 3-fluoropropyl, 1,1-difluoropropyl, 1,2-difluoropropyl, 1,3-difluoropropyl, 2,2-difluoropropyl, 2,3-difluoropropyl, 3,3-difluoropropyl, 1,1,2-trifluoropropyl, 1,1,3-trifluoropropyl, 1,2,3-trifluoropropyl, 1,3,3-trifluoropropyl, 2,2,3-trifluoropropyl, 2,3,3-trifluoropropyl, 3,3,3-trifluoropropyl, 1,1,2,2-tetrafluoropropyl, 1,1,2,3-tetrafluoropropyl, 1,2,2,3-tetrafluoropropyl, 1,3,3,3-tetrafluoropropyl, 2,2,3,3-tetrafluoropropyl, 2,3,3,3-tetrafluoropropyl, 1,1,2,2,3-pentafluoropropyl, 1,2,2,3,3-pentafluoropropyl, 1,1,3,3,3-pentafluoropropyl, 1,2,3,3,3-pentafluoropropyl, 2,2,3,3,3-pentafluoropropyl and heptafluoropropyl groups.

The fluorocycloalkyl group is not particularly limited, provided that it is a cycloalkyl group in which at least one hydrogen atom on a carbon atom is substituted with a fluorine atom. Examples thereof include 1-fluorocyclopropyl, 2-fluorocyclopropyl, 2,2-difluorocyclopropyl, 2,2,3,3-tetrafluorocyclopropyl, pentafluorocyclopropyl, 2,2-difluorocyclobutyl, 2,2,3,3-tetrafluorocyclobutyl, 2,2,3,3,4,4-hexafluorocyclobutyl, heptafluorocyclobutyl, 1-fluorocyclopentyl, 3-fluorocyclopentyl, 3,3-difluorocyclopentyl, 3,3,4,4-tetrafluorocyclopentyl, nonafluorocyclopentyl, 1-fluorocyclohexyl, 2-fluorocyclohexyl, 4-fluorocyclohexyl, 4,4-difluorocyclohexyl, 2,2,3,3-tetrafluorocyclohexyl, 2,3,4,5,6-pentafluorocyclohexyl and undecafluorocyclohexyl groups.

The fluorobicycloalkyl group is not particularly limited, provided that it is a bicycloalkyl group in which at least one hydrogen atom on a carbon atom is substituted with a fluorine atom. Examples thereof include 3-fluorobicyclo[1.1.0]butan-1-yl, 2,2,4,4-tetrafluorobicyclo[1.1.0]butan-1-yl, pentafluorobicyclo[1.1.0]butan-1-yl, 3-fluorobicyclo[1.1.1]pentan-1-yl, 2,2,4,4,5-pentafluorobicyclo[1.1.1]pentan-1-yl, 2,2,4,4,5,5-hexafluorobicyclo[1.1.1]pentan-1-yl, 5-fluorobicyclo[3.1.0]hexan-6-yl, 6-fluorobicyclo[3.1.0]hexan-6-yl, 6,6-difluorobicyclo[3.1.0]hexan-2-yl, 2,2,3,3,5,5,6,6-octafluorobicyclo[2.2.0]hexan-1-yl, 1-fluorobicyclo[2.2.1]heptan-2-yl, 3-fluorobicyclo[2.2.1]heptan-2-yl, 4-fluorobicyclo[2.2.1]heptan-1-yl, 5-fluorobicyclo[3.1.1]heptan-1-yl, 1,3,3,4,5,5,6,6,7,7-decafluorobicyclo[2.2.1]heptan-2-yl, undecafluorobicyclo[2.2.1]heptan-2-yl, 3-fluorobicyclo[2.2.2]octan-1-yl and 4-fluorobicyclo[2.2.2]octan-1-yl groups.

The fluoroalkenyl group is not particularly limited, provided that it is an alkenyl group in which at least one hydrogen atom on a carbon atom is substituted with a fluorine atom. Examples thereof include 1-fluoroethenyl, 2-fluoroethenyl, 1,2-difluoroethenyl, 1,2,2-trifluoroethenyl, 2,3,3-trifluoro-1-propenyl, 3,3,3-trifluoro-1-propenyl, 2,3,3,3-tetrafluoro-1-propenyl, pentafluoro-1-propenyl, 1-fluoro-2-propenyl, 1,1-difluoro 2-propenyl, 2,3-difluoro-2-propenyl, 3,3-difluoro-2-propenyl, 2,3,3-trifluoro-2-propenyl, 1,2,3,3-tetrafluoro-2-propenyl and pentafluoro-2-propenyl groups.

The fluoroalkynyl group is not particularly limited, provided that it is an alkynyl group in which at least one hydrogen atom on a carbon atom is substituted with a fluorine atom. Examples thereof include fluoroethynyl, 3-fluoro-1-propynyl, 3,3-difluoro-1-propynyl, 3,3,3-trifluoro-1-propynyl, 1-fluoro-2-propynyl and 1,1-difluoro-2-propynyl groups.

The fluoroaryl group is not particularly limited, provided that it is an aryl group in which at least one hydrogen atom on a carbon atom is substituted with a fluorine atom. Examples thereof include 2-fluorophenyl, 3-fluorophenyl, 4-fluorophenyl, 2,3-difluorophenyl, 2,4 difluorophenyl, 2,5-difluorophenyl, 2,6-difluorophenyl, 3,4-difluorophenyl, 3,5-difluorophenyl, 2,3,4-trifluorophenyl, 2,3,5-trifluorophenyl, 2,3,6-trifluorophenyl, 2,4,5-trifluorophenyl, 2,4,6-trifluorophenyl, 3,4,5-trifluorophenyl, 2,3,4,5-tetrafluorophenyl, 2,3,4,6-tetrafluorophenyl, 2,3,5,6-tetrafluorophenyl, pentafluorophenyl, 2-fluoro-1-naphthyl, 3-fluoro-1-naphthyl, 4-fluoro-1-naphthyl, 6-fluoro-1-naphthyl, 7-fluoro-1-naphthyl, 8-fluoro-1-naphthyl, 4,5-difluoro-1-naphthyl, 5,7-difluoro-1-naphthyl, 5,8-difluoro-1-naphthyl, 5,6,7,8-tetrafluoro-1-naphthyl, heptafluoro-1-naphthyl, 1-fluoro-2-naphthyl, 5-fluoro-2-naphthyl, 6-fluoro-2-naphthyl, 7-fluoro-2-naphthyl, 5,7-difluoro-2-naphthyl and heptafluoro-2-naphthyl groups.

The fluoroaryl group, taking into account the balance between, for example, the solubility of the fluorine atom-containing oligoaniline derivative in organic solvents, the charge transportability of the fluorine atom-containing oligoaniline derivative and the availability of starting materials for the fluorine atom-containing oligoaniline derivative, is preferably a phenyl group which is substituted with three or more fluorine atoms and which is optionally substituted with a cyano group, a chlorine atom, a bromine atom, an iodine atom, a nitro group, an alkyl group of 1 to 20 carbon atoms, a fluoroalkyl group of 1 to 20 carbon atoms or a fluoroalkoxy group of 1 to 20 carbon atoms.

The fluoroalkoxy group is not particularly limited, provided that it is an alkoxy group in which at least one hydrogen atom on a carbon atom is substituted with a fluorine atom. Examples thereof include fluoromethoxy, difluoromethoxy, trifluoromethoxy, 1-fluoroethoxy, 2-fluoroethoxy, 1,2-difluoroethoxy, 1,1-difluoroethoxy, 2,2-difluoroethoxy, 1,1,2-trifluoroethoxy, 1,2,2-trifluoroethoxy, 2,2,2-trifluoroethoxy, 1,1,2,2-tetrafluoroethoxy, 1,2,2,2-tetrafluoroethoxy, 1,1,2,2,2-pentafluoroethoxy, 1-fluoropropoxy, 2-fluoropropoxy, 3-fluoropropoxy, 1,1-difluoropropoxy, 1,2-difluoropropoxy, 1,3-difluoropropoxy, 2,2-difluoropropoxy, 2,3-difluoropropoxy, 3,3-difluoropropoxy, 1,1,2-trifluoropropoxy, 1,1,3-trifluoropropoxy, 1,2,3-trifluoropropoxy, 1,3,3-trifluoropropoxy, 2,2,3-trifluoropropoxy, 2,3,3-trifluoropropoxy, 3,3,3-trifluoropropoxy, 1,1,2,2-tetrafluoropropoxy, 1,1,2,3 -tetrafluoropropoxy, 1,2,2,3 -tetrafluoropropoxy, 1,3,3,3 -tetrafluoropropoxy, 2,2,3,3-tetrafluoropropoxy, 2,3,3,3-tetrafluoropropoxy, 1,1,2,2,3-pentafluoropropoxy, 1,2,2,3,3-pentafluoropropoxy, 1,1,3,3,3-pentafluoropropoxy, 1,2,3,3,3-pentafluoropropoxy, 2,2,3,3,3-pentafluoropropoxy and heptafluoropropoxy groups.

The substituted aryl group is not particularly limited, provided that it is an aryl group in which at least one hydrogen atom on a carbon atom is substituted with a fluoroalkyl group of 1 to 20 carbon atoms, a fluorocycloalkyl group of 3 to 20 carbon atoms, a fluorobicycloalkyl group of 4 to 20 carbon atoms, a fluoroalkenyl group of 2 to 20 carbon atoms or a fluoroalkynyl group of 2 to 20 carbon atoms. Examples thereof include 2-(trifluoromethyl)phenyl, 3-(trifluoromethyl)phenyl, 4-(trifluoromethyl)phenyl, 4-ethoxy-3-(trifluoromethyl)phenyl, 3-fluoro-4-trifluoromethylphenyl, 4-fluoro-3-trifluoromethylphenyl, 4-fluoro-2-trifluoromethylphenyl, 2-fluoro-5-(trifluoromethyl)phenyl, 3-fluoro-5-(trifluoromethyl)phenyl, 3,5-di(trifluoromethyl)phenyl, 2,4,6-tri(trifluoromethyl)phenyl, 4-(pentafluoroethyl)phenyl, 4-(3,3,3-trifluoropropyl)phenyl, 2,3,5,6-tetrafluoro-4-trifluoromethylphenyl, 4-(perfluorovinyl)phenyl, 4-(perfluoropropenyl)phenyl and 4-(perfluorobutenyl)phenyl groups.

The substituted aryl group, taking into account the balance between the solubility of the fluorine atom-containing oligoaniline derivative in organic solvents and the availability of starting materials for the fluorine atom-containing oligoaniline derivative, is preferably a phenyl group which is substituted with a fluorocycloalkyl group of 3 to 20 carbon atoms, a fluorobicycloalkyl group of 4 to 20 carbon atoms, a fluoroalkenyl group of 2 to 20 carbon atoms or a fluoroalkynyl group of 2 to 20 carbon atoms and which may also be substituted with a cyano group, a halogen atom or a fluoroalkoxy group of 1 to 20 carbon atoms (which phenyl group is also referred to below, for the sake of convenience, as a "substituted phenyl group"); more preferably a phenyl group substituted with from one to three trifluoromethyl groups; and even more preferably a p-trifluoromethylphenyl group.

The fluoroaralkyl group is not particularly limited, provided it is an aralkyl group in which at least one hydrogen atom on a carbon atom is substituted with a fluorine atom. Examples include 2-fluorobenzyl, 3-fluorobenzyl, 4-fluorobenzyl, 2,3-difluorobenzyl, 2,4-difluorobenzyl, 2,5-difluorobenzyl, 2,6-difluorobenzyl, 3,4-difluorobenzyl, 3,5-difluorobenzyl, 2,3,4-trifluorobenzyl, 2,3,5-trifluorobenzyl, 2,3,6-trifluorobenzyl, 2,4,5-trifluorobenzyl, 2,4,6-trifluorobenzyl, 2,3,4,5-tetrafluorobenzyl, 2,3,4,6-tetrafluorobenzyl, 2,3,5,6-tetraflurobenzyl and 2,3,4,5,6-pentafluorobenzyl groups.

The substituted aralkyl group is not particularly limited, provided that it is an aralkyl group in which at least one hydrogen atom on a carbon atom is substituted with a fluoroalkyl group of 1 to 20 carbon atoms, a fluorocycloalkyl group of 3 to 20 carbon atoms, a fluorobicycloalkyl group of 4 to 20 carbon atoms, a fluoroalkenyl group of 2 to 20 carbon atoms or a fluoroalkynyl group of 2 to 20 carbon atoms. Examples thereof include 2-trifluoromethylbenzyl, 3-trifluoromethylbenzyl, 4-trifluoromethylbenzyl, 2,4-di(trifluoromethyl)benzyl, 2,5-di(trifluoromethyl)benzyl, 2,6-di(trifluoromethyl)benzyl, 3,5-di(trifluoromethyl)benzyl and 2,4,6-tri(trifluoromethyl)benzyl groups.

Of these, Ar^{F} is preferably the above fluoroalkyl group of 1 to 20 carbon atoms which is optionally substituted, the above fluoroaryl group of 6 to 20 carbon atoms which is optionally substituted or the above substituted aryl group; more preferably the above fluoroaryl group of 6 to 20 carbon atoms which is optionally substituted or the above substituted aryl group; even more preferably the above fluorophenyl group optionally substituted or the above substituted phenyl group; and still more preferably the above trifluorophenyl group optionally substituted, the above tetrafluorophenyl group optionally substituted, the above pentafluorophenyl group optionally substituted or a phenyl group substituted with from one to three trifluoromethyl groups.

In formula (A3), the subscript r is an integer from 1 to 20. However, from the standpoint of the solubility of the fluorine atom-containing oligoaniline derivative in solvents, r is preferably 10 or less, more preferably 8 or less, even more preferably 5 or less, and still more preferably 4 or less. From the standpoint of increasing the charge transportability of the fluorine atom-containing oligoaniline derivative, the subscript r is preferably 2 or more, and more preferably 3 or more. Taking into account the balance between solubility and charge transportability, the subscript r is most preferably 3.

The fluorine atom-containing oligoaniline derivative of formula (A3) can be produced according to, for example, the method described in WO 2016/117521 or WO 2016/190326.

As the above aniline derivative, the aniline derivatives of formula (A4) below can be used.

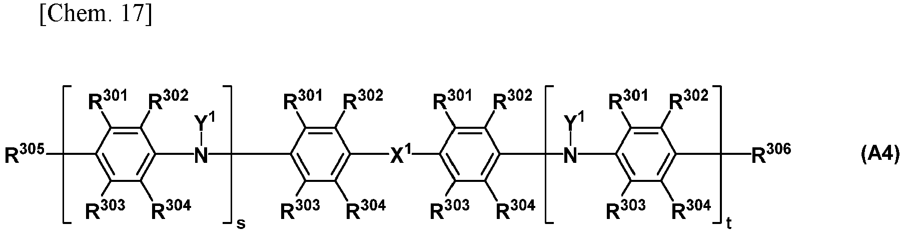

In formula (A4), X¹ is -NY¹-, -O-, -S-, -(CR³⁰⁷R³⁰⁸)_{L}- or a single bond. When s or t is 0, X¹ is -NY¹-.

Each Y¹ in formula (A4) is independently a hydrogen atom, or an alkyl group of 1 to 20 carbon atoms, alkenyl group of 2 to 20 carbon atoms or alkynyl group of 2 to 20 carbon atoms which is optionally substituted with Z²¹, or an aryl group of 6 to 20 carbon atoms or heteroaryl group of 2 to 20 carbon atoms which is optionally substituted with Z²².

Specific examples of the alkyl group of 1 to 20 carbon atoms, alkenyl group of 2 to 20 carbon atoms, alkynyl group of 2 to 20 carbon atoms, aryl group of 6 to 20 carbon atoms and heteroaryl group of 2 to 20 carbon atoms include the same as those mentioned above.

R³⁰⁷ and R³⁰⁸ are each independently a hydrogen atom, a chlorine atom, a bromine atom, an iodine atom, a nitro group, a cyano group, an amino group, an aldehyde group, a hydroxyl group, a thiol group, a sulfonic acid group, or a carboxyl group, or an alkyl group of 1 to 20 carbon atoms, alkenyl group of 2 to 20 carbon atoms or alkynyl group of 2 to 20 carbon atoms which is optionally substituted with Z²¹, or an aryl group of 6 to 20 carbon atoms or heteroaryl group of 2 to 20 carbon atoms which is optionally substituted with Z²², or -NHY², -NY³Y⁴, -C(O)Y⁵, -OY⁶, -SY⁷, -SO₃Y⁸, -C(O)OY⁹, -OC(O)Y¹⁰, -C(O)NHY¹¹ or -C(O)NY¹²Y¹³.

Y² to Y¹³ are each independently an alkyl group of 1 to 20 carbon atoms, alkenyl group of 2 to 20 carbon atoms or alkynyl group of 2 to 20 carbon atoms which is optionally substituted with Z²¹, or an aryl group of 6 to 20 carbon atoms or heteroaryl group of 2 to 20 carbon atoms which is optionally substituted with Z²².

Z²¹ is a chlorine atom, a bromine atom, an iodine atom, a nitro group, a cyano group, an amino group, an aldehyde group, a hydroxy group, a thiol group, a sulfonic acid group or a carboxy group, or an aryl group of 6 to 20 carbon atoms or a heteroaryl group of 2 to 20 carbon atoms which is optionally substituted with Z²³.

Z²² is a chlorine atom, a bromine atom, an iodine atom, a nitro group, a cyano group, an amino group, an aldehyde group, a hydroxy group, a thiol group, a sulfonic acid group, a carboxy group, or an alkyl group an aryl group of 1 to 20 carbon atoms, an alkenyl group of 2 to 20 carbon atoms or an alkynyl group of 2 to 20 carbon atoms which is optionally substituted with Z²³.

Z²³ is a chlorine atom, a bromine atom, an iodine atom, a nitro group, a cyano group, an amino group, an aldehyde group, a hydroxy group, a thiol group, a sulfonic acid group or a carboxy group.

Examples of the alkyl group, alkenyl group, alkynyl group, aryl group and heteroaryl group represented by R³⁰⁷, R³⁰⁸ and Y² to Y¹³ include the same as those mentioned above.

Of these, R³⁰⁷ and R³⁰⁸ are preferably hydrogen atoms or alkyl groups of 1 to 20 carbon atoms which are optionally substituted with Z²¹, more preferably hydrogen atoms or methyl groups which are optionally substituted with Z²¹, and most preferably both hydrogen atoms.

L, which represents the number of divalent groups of the formula -(CR³⁰⁷R³⁰⁸)-, is an integer from 1 to 20, preferably from 1 to 10, more preferably from 1 to 5, even more preferably 1 or 2, and most preferably 1. When L is 2 or more, the plurality of R³⁰⁷ groups may be mutually the same or different, and the plurality of R³⁰⁸ may be mutually the same or different.

In particular, X¹ is preferably -NY¹- or a single bond. Y¹ is preferably a hydrogen atom or an alkyl group of 1 to 20 carbon atoms which is optionally substituted with Z²¹, more preferably a hydrogen atom or methyl group which is optionally substituted with Z²¹, and most preferably a hydrogen atom.

In formula (A4), R³⁰¹ to R³⁰⁶ are each independently a hydrogen atom, a chlorine atom, a bromine atom, an iodine atom, a nitro group, a cyano group, an amino group, an aldehyde group, a hydroxyl group, a thiol group, a sulfonic acid group, a carboxyl group, or an alkyl group of 1 to 20 carbon atoms, alkenyl group of 2 to 20 carbon atoms or alkynyl group of 2 to 20 carbon atoms which is optionally substituted with Z²¹, an aryl group of 6 to 20 carbon atoms or heteroaryl group of 2 to 20 carbon atoms which is optionally substituted with Z²², or -NHY², -NY³Y⁴, -C(O)Y⁵, -OY⁶, -SY⁷, -SO₃Y⁸, -C(O)OY⁹, -OC(O)Y¹⁰, -C(O)NHY¹¹ or -C(O)NY¹²Y¹³ (Y² to Y¹³ are the same as described above). Examples of the alkyl group, alkenyl group, alkynyl group, aryl group and heteroaryl group include the same as those mentioned above.

In particular, in formula (A4), R³⁰¹ to R³⁰⁴ are each preferably a hydrogen atom, a halogen atom, an alkyl group of 1 to 10 carbon atoms which is optionally substituted with Z²¹, or an aryl group of 6 to 14 carbon atoms which is optionally substituted with Z²²; more preferably a hydrogen atom or an alkyl group of 1 to 10 carbon atoms; and most preferably are all hydrogen atoms.

R³⁰⁵ and R³⁰⁶ are each preferably a hydrogen atom, a chlorine atom, a bromine atom, an iodine atom, an alkyl group of 1 to 10 carbon atoms which is optionally substituted with Z²¹, an aryl group of 6 to 14 carbon atoms which is optionally substituted with Z²², or a diphenylamino group optionally substituted with Z²² (i.e. the phenyl group -NY³Y⁴ wherein Y³ and Y⁴ are optionally substituted with Z²²); are more preferably a hydrogen atom or a diphenylamino group; and are even more preferably both hydrogen atoms or both diphenylamino groups.

Of these, a combination in which R³⁰¹ to R³⁰⁴ are each preferably a hydrogen atom or an alkyl group of 1 to 10 carbon atoms, R³⁰⁵ and R³⁰⁶ are each a hydrogen atom or a diphenylamino group, X¹ is -NY¹- or a single bond and Y¹ is a hydrogen atom or a methyl group is preferred; and a combination in which R³⁰¹ to R³⁰⁴ are each a hydrogen atom, R³⁰⁵ and R³⁰⁶ are both hydrogen atoms or diphenylamino groups, and X¹ is -NH- or a single bond is more preferred.

In formula (A4), s and t are each independently an integer of 0 or more and together satisfy the condition 1 ≤ s+t ≤ 20. Taking into account the balance between the charge transportability of the resulting thin film and the solubility of the aniline derivative, they preferably satisfy the condition 2 ≤ s+t ≤ 8, more preferably satisfy the condition 2 ≤ s+t ≤ 6, and even more preferably satisfy the condition 2 ≤ s+t ≤ 4.

In Y¹ to Y¹³ and R³⁰¹ to R³⁰⁸, Z²¹ is preferably a chlorine atom, a bromine atom, an iodine atom or an aryl group of 6 to 20 carbon atoms which is optionally substituted with Z²³; more preferably a chlorine atom, a bromine atom, an iodine atom or a phenyl group which is optionally substituted with Z²³; and most preferably does not exist (i.e., is non-substituting).

Z²² is preferably a chlorine atom, a bromine atom, an iodine atom or an alkyl group of 1 to 20 carbon atoms which is optionally substituted with Z²³, more preferably a chlorine atom, a bromine atom, an iodine atom or an alkyl group of 1 to 4 carbon atoms which is optionally substituted with Z²³; and most preferably does not exist (i.e., is non-substituting).

Z²³ is preferably a chlorine atom, a bromine atom or an iodine atom; and most preferably does not exist (i.e., is non-substituting).

In Y¹ to Y¹³ and R³⁰¹ to R³⁰⁸, the number of carbon atoms on the alkyl, alkenyl and alkynyl groups is preferably 10 or less, more preferably 6 or less, and even more preferably 4 or less. The number of carbon atoms on the aryl and heteroaryl groups is preferably 14 or less, more preferably 10 or less, and even more preferably 6 or less.

The aniline derivative of formula (A4) can be produced according to, for example, the method described in Bulletin of Chemical Society of Japan, 67, pp. 1749-1752 (1994); Synthetic Metals, 84, pp. 119-120 (1997); Thin Solid Films, 520(24), pp. 7157-7163 (2012); and WO 2008/032617, WO 2008/032616, WO 2008/129947 and WO 2013/084664.

Specific examples of the aniline derivative of formula (A4) include, but are not limited to, those of the following formulae (A4-1) to (A4-12). In following formulae, "DPA" stands for a diphenylamino group, "Ph" stands for a phenyl group, and "TPA" stands for a p-(diphenylamino)phenyl group.

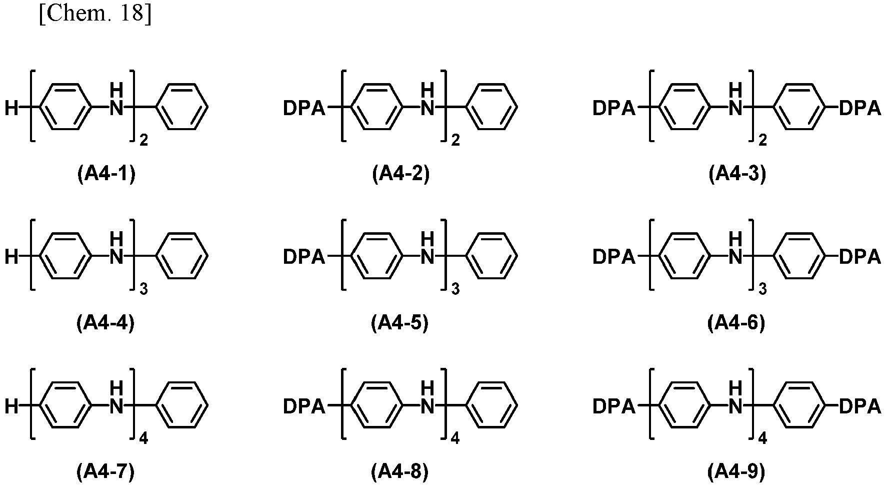

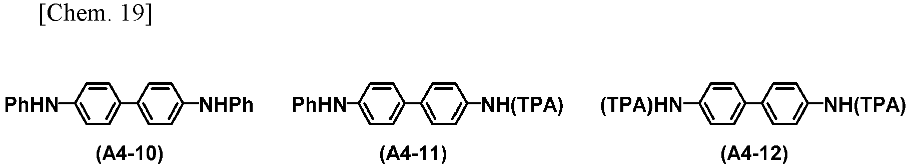

The charge-transporting substances may be used singly, or in combination of two or more thereof.

### [Organic Solvent]

A high-solvency solvent capable of dissolving well the above aniline derivatives and sulfonic acid ester compounds may be used as the organic solvent employed when preparing the charge-transporting varnish of the invention. To dissolve an unesterified sulfonic acid compound, it is necessary that at least one highly polar solvent be included. By contrast, it is possible to dissolve the above sulfonic acid ester compounds in a solvent regardless of the polarity of the solvent. In the invention, a low-polarity solvent is defined as a solvent having a dielectric constant at a frequency of 100 kHz that is less than 7, and a high-polarity solvent is defined as a solvent having a dielectric constant at a frequency of 100 kHz that is 7 or more.

Examples of low-polarity solvents include
chlorinated solvents such as chloroform and chlorobenzene;
aromatic hydrocarbon solvents such as toluene, xylene, tetralin, cyclohexylbenzene and decylbenzene;
aliphatic alcohol solvents such as 1-octanol, 1-nonanol and 1-decanol;
ether solvents such as tetrahydrofuran, dioxane, anisole, 4-methoxytoluene, 3-phenoxytoluene, dibenzyl ether, diethylene glycol dimethyl ether, diethylene glycol butyl methyl ether, triethylene glycol dimethyl ether and triethylene glycol butyl methyl ether; and
ester solvents such as methyl benzoate, ethyl benzoate, butyl benzoate, isoamyl benzoate, bis(2-ethylhexyl) phthalate, dibutyl maleate, dibutyl oxalate, hexyl acetate, diethylene glycol monoethyl ether acetate and diethylene glycol monobutyl ether acetate.

Examples of high-polarity solvents include
amide solvents such as N,N-dimethylformamide, N,N-dimethylacetamide, N,N-dimethylisobutyramide, N-methylpyrrolidone and 1,3-dimethyl-2-imidazolidinone;
ketone solvents such as ethyl methyl ketone, isophorone and cyclohexanone;
cyano solvents such as acetonitrile and 3-methoxypropionitrile;
polyhydric alcohol solvents such as ethylene glycol, diethylene glycol, triethylene glycol, dipropylene glycol, 1,3-butanediol and 2,3-butanediol;
monohydric alcohol solvents other than aliphatic alcohols, such as
diethylene glycol monomethyl ether, diethylene glycol monophenyl ether, triethylene glycol monomethyl ether, dipropylene glycol monomethyl ether, benzyl alcohol, 2-phenoxyethanol, 2-benzyloxyethanol, 3-phenoxybenzyl alcohol and tetrahydrofurfuryl alcohol; and
sulfoxide solvents such as dimethylsulfoxide. Depending on the intended use, these solvents may be used singly, or in admixture of two or more thereof.

It is preferable for all the charge-transporting substances to be in a completely dissolved or uniformly dispersed state in the above solvent, and more preferable for them to be completely dissolved.

Examples of the method for preparing a charge-transporting varnish include, but are not limited to, a method in which a charge-transporting substance, an electron-accepting substance precursor and the like are added to a solvent in any order or at the same time. When there are a plurality of organic solvents, a charge-transporting substance, an electron-accepting substance precursor and the like may be dissolved in one solvent, followed by adding another solvent thereto, or a charge-transporting substance, a dopant and the like may be dissolved in a mixed solvent of a plurality of organic solvents in order or at the same time.

From the standpoint of reproducibly obtaining thin films having a higher flatness, it is desirable for the charge-transporting varnish, the electron-accepting substance precursor and the like to be obtained by dissolving the charge-transporting substance in the organic solvent and subsequently filtering the solution using a submicron-order filter or the like.

The solids concentration in the varnish of the invention, from the standpoint of ensuring a sufficient film thickness while minimizing deposition of the charge-transporting substance, is generally from about 0.1 to about 20% by weight, and preferably from 0.5 to 10% by weight. As used herein, the "solid" refers to the constituents which are contained in the varnish and which do not include solvents. The viscosity of the inventive varnish is generally from 1 to 50 mPa·s at 25°C.

The content of the electron-accepting substance precursor within these solids, expressed as a molar ratio with respect to unity (1) for the charge-transporting substance, is preferably from about 0.01 to about 20, and more preferably from about 0.05 to about 15.

The charge-transporting varnish of the present invention may further contain an organic silane compound. Examples of the organic silane compound include dialkoxysilane compounds, trialkoxysilane compounds and tetraalkoxysilane compounds. In particular, the organic silane compound is preferably a dialkoxysilane compound or a trialkoxysilane compound, and more preferably a trialkoxysilane compound. The organic silane compounds may be used singly, or in combination of two or more thereof.

The content of the organic silane compound is typically from about 0.1 to 50% by weight based on the total mass of the charge-transporting substance and the dopant. Taking into account the suppression of deterioration of charge transportability of the resulting thin film and the enhancement of the hole-injecting ability into layers laminated so as to contact the hole-injecting layer on a side opposite to the anode, such as a hole-transporting layer and a light-emitting layer, the content of the organic silane compound is preferably from about 0.5 to 40% by weight, more preferably from about 0.8 to 30% by weight, still more preferably from about 1 to 20% by weight.

### [Charge-Transporting Thin Film]

A charge-transporting thin film can be formed on a substrate by applying the charge-transporting varnish of the invention onto the substrate and drying the applied varnish.

Examples of methods for applying the varnish include, but are not limited to, dipping, spin coating, transfer printing, roll coating, brush coating, inkjet coating, spraying and slit coating. It is preferable for the viscosity and surface tension of the varnish to be adjusted according to the method of application.

When using the varnish of the invention, the liquid film drying conditions are not particularly limited; one example is heating and firing on a hot plate. A dry film can be obtained by heating and firing in a temperature range of generally from about 100 to about 260°C for a period of from about 1 minute to about 1 hour. The firing atmosphere is not particularly limited.

The thickness of the charge-transporting thin film is not particularly limited. However, when the thin film is to be used as a functional layer in an organic EL device, a film thickness of from 5 to 200 nm is preferred. Methods for changing the film thickness include, for example, changing the solids concentration in the varnish and changing the amount of solution on the substrate at the time of application.

### [Organic EL Device]

The organic EL device of the invention has a pair of electrodes and additionally has, between these electrodes, the above-described charge-transporting thin film of the invention.

Typical organic EL device configurations include, but are not limited to, the following configurations (a) to (f). In these configurations, where necessary, an electron-blocking layer or the like may be provided between the light-emitting layer and the anode, and a hole-blocking layer or the like may be provided between the light-emitting layer and the cathode. Alternatively, the hole-injecting layer, hole-transporting layer or hole-injecting-and-transporting layer may also have the function of an electron-blocking layer or the like; and the electron-injecting layer, electron-transporting layer or electron-injecting-and-transporting layer may also have the function of a hole-blocking layer or the like.
(a) anode/hole-injecting layer/hole-transporting layer/light-emitting layer/electron-transporting layer/electron-injecting layer/cathode
(b) anode/hole-injecting layer/hole-transporting layer/light-emitting layer/electron-inj ecting-and-transporting layer/cathode
(c) anode/hole-inj ecting-and-transporting layer/light-emitting layer/electron-transporting layer/electron-injecting layer/cathode
(d) anode/hole-inj ecting-and-transporting layer/light-emitting layer/electron-inj ecting-and-transporting layer/cathode
(e) anode/hole-injecting layer/hole-transporting layer/light-emitting layer/cathode
(f) anode/hole-inj ecting-and-transporting layer/light-emitting layer/cathode

As used herein, "hole-inj ecting layer," "hole-transporting layer" and "hole injecting-and-transporting layer" refer to layers which are formed between the light emitting layer and the anode and which have the function of transporting holes from the anode to the light-emitting layer. When only one layer of hole-transporting material is provided between the light-emitting layer and the anode, this is a "hole injecting and transporting layer"; when two or more layers of hole-transporting material are provided between the light-emitting layer and the anode, the layer that is closer to the anode is a "hole-injecting layer" and the other layer is a "hole-transporting layer." In particular, thin films having not only an excellent ability to accept holes from the anode but also an excellent ability to inject holes into, respectively, the hole-transporting layer and the light-emitting layer may be used as the hole-injecting layer and the hole injecting and-transporting layer.

The "electron-injecting layer," "electron-transporting layer" and "electron injecting-and-transporting layer" refer to layers which are formed between the light-emitting layer and the cathode and which have the function of transporting electrons from the cathode to the light-emitting layer. When only one layer of electron-transporting material is provided between the light-emitting layer and the cathode, this is an "electron injecting-and-transporting layer"; when two or more layers of electron transporting material are provided between the light-emitting layer and the cathode, the layer that is closer to the cathode is an "electron-injecting layer" and the other layer is an "electron-transporting layer."

The "light-emitting layer" is an organic layer having a light-emitting function. When a doping system is used, this layer includes a host material and a dopant material. The function of the host material is primarily to promote the recombination of electrons and holes and to confine the resulting excitons within the light-emitting layer. The function of the dopant material is to cause the excitons obtained by recombination to efficiently luminesce. In the case of phosphorescent devices, the host material functions primarily to confine within the light-emitting layer the excitons generated by the dopant.

The materials and method employed to produce an organic EL device using the charge-transporting varnish of the invention are exemplified by, but not limited to, those described below.

The electrode substrate to be used is preferably cleaned beforehand by liquid washing with, for example, a cleaning agent, alcohol or pure water. For example, when the electrode substrate is an anode substrate, it is preferably subjected to surface treatment such as UV/ozone treatment or oxygen-plasma treatment just prior to use. However, surface treatment need not be carried out in cases where the anode material contains an organic substance as a principal component.

An example is described below of a method for producing the organic EL device of the invention in which a thin-film obtained from the charge-transporting varnish of the invention serves as the hole-injecting layer.

Using the above-described method, a hole-injecting layer is formed on an electrode by applying the charge-transporting varnish of the invention onto an anode substrate and then firing the applied varnish. A hole-transporting layer, a light-emitting layer, an electron-transporting layer, an electron-injecting layer and a cathode are provided in this order on the hole-injecting layer. The hole-transporting layer, light-emitting layer, electron-transporting layer and electron-injecting layer may be formed by either a vapor deposition process or a coating process (wet process), depending on the properties of the material used.

Illustrative examples of anode materials include transparent electrodes such as indium-tin oxide (ITO) and indium-zinc oxide (IZO), and metal anodes made of a metal such as aluminum or an alloy of such a metal. An anode material on which planarizing treatment has been carried out is preferred. Use can also be made of polythiophene derivatives and polyaniline derivatives having a high charge transportability.

Examples of other metals that may make up the metal anode include, but are not limited to, scandium, titanium, vanadium, chromium, manganese, iron, cobalt, nickel, copper, zinc, gallium, yttrium, zirconium, niobium, molybdenum, ruthenium, rhodium, palladium, cadmium, indium, scandium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, bismuth, and alloys thereof.

Specific examples of hole-transporting layer-forming materials include the following hole-transporting low-molecular-weight materials: triarylamines such as (triphenylamine) dimer derivatives, [(triphenylamine) dimer] spirodimer, N,N'-bis(naphthalen-1-yl)-N,N'-bis(phenyl)benzidine (α-NPD), N,N'-bis(naphthalen-2-yl)-N,N'-bis(phenyl)benzidine, N,N'-bis(3-methylphenyl)-N,N'-bis(phenyl)benzidine, N,N'-bis(3-methylphenyl)-N,N'-bis(phenyl)-9,9-spirobifluorene, N,N'-bis(naphthalen-1-yl)-N,N'-bis(phenyl)-9,9-spirobifluorene, N,N'-bis(3-methylphenyl)-N,N'-bis(phenyl)-9,9-dimethylfluorene, N,N'-bis(naphthalen-1-yl)-N,N'-bis(phenyl)-9,9-dimethylfluorene, N,N'-bis(3-methylphenyl)-N,N'-bis(phenyl)-9,9-diphenylfluorene, N,N'-bis(naphthalen-1-yl)-N,N'-bis(phenyl)-9,9-diphenylfluorene, N,N'-bis(naphthalen-1-yl)-N,N'-bis(phenyl)-2,2'-dimethylbenzidine, 2,2',7,7'-tetrakis(N,N-diphenylamino)-9,9-spirobifluorene, 9,9-bis[4-(N,N-bis-biphenyl-4-ylamino)phenyl]-9H-fluorene, 9,9-bis[4-(N,N-bisnaphthalen-2-ylamino)phenyl]-9H-fluorene, 9,9-bis[4-(N-naphthalen-1-yl-N-phenylamino)phenyl]-9H-fluorene, 2,2',7,7'-tetrakis[N-naphthalenyl(phenyl)amino]-9,9-spirobifluorene, N,N'-bis(phenanthren-9-yl)-N,N'-bis(phenyl)benzidine, 2,2'-bis[N,N-bis(biphenyl-4-yl)amino]-9,9-spirobifluorene, 2,2'-bis(N,N-diphenylamino)-9,9-spirobifluorene, di[4-(N,N-di(p-tolyl)amino)phenyl]cyclohexane, 2,2',7,7' -tetra(N,N-di(p-tolyl))amino-9,9-spirobifluorene, N,N,N',N'-tetra-naphthalen-2-yl-benzidine, N,N,N',N'-tetra(3-methylphenyl)-3,3'-dimethylbenzidine, N,N'-di(naphthalenyl)-N,N'-di(naphthalen-2-yl)benzidine, N,N,N',N'-tetra(naphthalenyl)benzidine, N,N'-di(naphthalen-2-yl)-N,N'-diphenylbenzidine-1-4-diamine, N¹,N⁴-diphenyl-N¹,N⁴-di(m-tolyl)benzene-1,4-diamine, N²,N²,N⁶,N⁶-tetraphenylnaphthalene-2,6-diamine, tris(4-(quinolin-8-yl)phenyl)amine, 2,2'-bis(3-(N,N-di(p-tolyl)amino)phenyl)biphenyl, 4,4',4"-tris[3-methylphenyl(phenyl)amino]triphenylamine (m-MTDATA) and 4,4',4"-tris[1-naphthyl(phenyl)amino]triphenylamine (1-TNATA); and oligothiophenes such as 5,5"-bis-{4-[bis(4-methylphenyl)amino]phenyl}-2,2':5',2"-terthiophene (BMA-3T).

Specific examples of light-emitting layer-forming materials include tris(8-quinolinolate) aluminum(III) (Alq₃), bis(8-quinolinolate) zinc(II) (Znq₂), bis(2-methyl-8-quinolinolate)-4-(p-phenylphenolate) aluminum(III) (BAlq), 4,4'-bis(2,2-diphenylvinyl)biphenyl, 9,10-di(naphthalen-2-yl)anthracene, 2-tert-butyl-9,10-di(naphthalen-2-yl)anthracene, 2,7-bis[9,9-di(4-methylphenyl)-fluoren-2-yl]-9,9-di(4-methylphenyl)fluorene, 2-methyl-9,10-bis(naphthalen-2-yl)anthracene, 2-(9,9-spirobifluoren-2-yl)-9,9-spirobifluorene, 2,7-bis(9,9-spirobifluoren-2-yl)-9,9-spirobifluorene, 2-[9,9-di(4-methylphenyl)-fluoren-2-yl]-9,9-di(4-methylphenyl)fluorene, 2,2'-dipyrenyl-9,9-spirobifluorene, 1,3,5-tris(pyren-1-yl)benzene, 9,9-bis[4-(pyrenyl)phenyl]-9H-fluorene, 2,2'-bi(9,10-diphenylanthracene), 2,7-dipyrenyl-9,9-spirobifluorene, 1,4-di(pyren-1-yl)benzene, 1,3-di(pyren-1-yl)benzene, 6,13-di(biphenyl-4-yl)pentacene, 3,9-di(naphthalen-2-yl)perylene, 3,10-di(naphthalen-2-yl)perylene, tris[4-(pyrenyl)-phenyl]amine, 10,10' -di(biphenyl-4-yl)-9,9' -bianthracene, N,N'-di(naphthalen-1-yl)-N,N'-diphenyl-[1,1':4',1":4",1'''-quaterphenyl]-4,4'''-diamine, 4,4'-di[10-(naphthalen-1-yl)anthracen-9-yl]biphenyl, dibenzo{[f,f']-4,4',7,7'-tetraphenyl}diindeno[1,2,3-cd:1',2',3'-lm]perylene, 1-(7-(9,9'-bianthracen-10-yl)-9,9-dimethyl-9H-fluoren-2-yl)pyrene, 1-(7-(9,9'-bianthracen-10-yl)-9,9-dihexyl-9H-fluoren-2-yl)pyrene, 1,3-bis(carbazol-9-yl)benzene, 1,3,5-tris(carbazol-9-yl)benzene, 4,4',4"-tris(carbazol-9-yl)triphenylamine, 4,4'-bis(carbazol-9-yl)biphenyl (CBP), 4,4'-bis(carbazol-9-yl)-2,2'-dimethylbiphenyl, 2,7-bis(carbazol-9-yl)-9,9-dimethylfluorene, 2,2',7,7'-tetrakis(carbazol-9-yl)-9,9-spirobifluorene, 2,7-bis(carbazol-9-yl)-9,9-di(p-tolyl)fluorene, 9,9-bis[4-(carbazol-9-yl)-phenyl]fluorene, 2,7-bis(carbazol-9-yl)-9,9-spirobifluorene, 1,4-bis(triphenylsilyl)benzene, 1,3-bis(triphenylsilyl)benzene, bis(4-N,N-diethylamino-2-methylphenyl)-4-methylphenylmethane, 2,7-bis(carbazol-9-yl)-9,9-dioctylfluorene, 4,4"-di(triphenylsilyl)-p-terphenyl, 4,4'-di(triphenylsilyl)biphenyl, 9-(4-tert-butylphenyl)-3,6-bis(triphenylsilyl)-9H-carbazole, 9-(4-tert-butylphenyl)-3,6-ditrityl-9H-carbazole, 9-(4-tert-butylphenyl)-3,6-bis(9-(4-methoxyphenyl)-9H-fluoren-9-yl)-9H-carbazole, 2,6-bis(3-(9H-carbazol-9-yl)phenyl)pyridine, triphenyl(4-(9-phenyl-9H-fluoren-9-yl)phenyl)silane, 9,9-dimethyl-N,N-diphenyl-7-(4-(1-phenyl-1H-benzo[d]imidazol-2-yl)phenyl-9H-fluoren-2-amine, 3,5-bis(3-(9H-carbazol-9-yl)phenyl)pyridine, 9,9-spirobifluoren-2-yl-diphenyl-phosphine oxide, 9,9'-(5-triphenylsilyl)-1,3-phenylene)bis(9H-carbazole), 3-(2,7-bis(diphenylphosphoryl)-9-phenyl-9H-fluoren-9-yl)-9-phenyl-9H-carbazole, 4,4,8,8,12,12-hexa(p-tolyl)-4H-8H-12H-12C-azadibenzo[cd,mn]pyrene, 4,7-di(9H-carbazol-9-yl)-1,10-phenanthroline, 2,2'-bis(4-(carbazol-9-yl)phenyl)biphenyl, 2,8-bis(diphenylphosphoryl)dibenzo[b,d]thiophene, bis(2-methylphenyl)diphenylsilane, bis[3,5-di(9H-carbazol-9-yl)phenyl]diphenylsilane, 3,6-bis(carbazol-9-yl)-9-(2-ethylhexyl)-9H-carbazole, 3-(diphenylphosphoryl)-9-(4-(diphenylphosphoryl)phenyl)-9H-carbazole and 3,6-bis[(3,5-diphenyl)phenyl]-9-phenylcarbazole. The light-emitting layer may be formed by the co-vapor deposition of these materials with a light-emitting dopant.

Specific examples of light-emitting dopants include 3-(2-benzothiazolyl)-7-(diethylamino)coumarin, 2,3,6,7-tetrahydro-1,1,7,7-tetramethyl-1H,5H,11H-10-(2-benzothiazolyl)quinolidino-[9,9a,1gh]coumarin, quinacridone, N,N'-dimethylquinacridone, tris(2-phenylpyridine) iridium(III) (Ir(ppy)₃), bis(2-phenylpyridine)(acetylacetonate) iridium(III) (Ir(ppy)₂(acac)), tris[2-(p-tolyl)pyridine] iridium(III) (Ir(mppy)₃), 9,10-bis[N,N-di(p-tolyl)amino]anthracene, 9,10-bis[phenyl(m-tolyl)amino]anthracene, bis[2-(2-hydroxyphenyl)benzothiazolate] zinc(II), N¹⁰,N¹⁰,N¹⁰,N¹⁰-tetra(p-tolyl)-9,9'-bianthracene-10,10' -diamine, N¹⁰,N¹⁰,N¹⁰,N¹⁰-tetraphenyl-9,9'-bianthracene-10,10' -diamine, N¹⁰,N¹⁰-diphenyl-N¹⁰,N¹⁰-dinaphthalenyl-9,9'-bianthracene-10,10' -diamine, 4,4'-bis(9-ethyl-3-carbazovinylene)-1,1'-biphenyl, perylene, 2,5,8,11-tetra-tert-butylperylene, 1,4-bis[2-(3-N-ethylcarbazolyl)vinyl]benzene, 4,4'-bis[4-(di-p-tolylamino)styryl]biphenyl, 4-(di-p-tolylamino)-4'-[(di-p-tolylamino)styryl]stilbene, bis[3,5-difluoro-2-(2-pyridyl)phenyl-(2-carboxypyridyl)] iridium(III), 4,4'-bis[4-(diphenylamino)styryl]biphenyl, bis(2,4-difluorophenylpyridinato)tetrakis(1-pyrazolyl)borate iridium(III), N,N'-bis(naphthalen-2-yl)-N,N'-bis(phenyl)-tris(9,9-dimethylfluorenylene), 2,7-bis{2-[phenyl(m-tolyl)amino]-9,9-dimethylfluoren-7-yl}-9,9-dimethylfluorene, N-(4-((E)-2-(6((E)-4-(diphenylamino)styryl)naphthalen-2-yl)vinyl)phenyl)-N-phenylbenzenamine, fac-iridium(III) tris(1-phenyl-3-methylbenzimidazolin-2-ylidene-C,C²), mer-iridium(III) tris(1-phenyl-3-methylbenzimidazolin-2-ylidene-C,C²), 2,7-bis[4-(diphenylamino)styryl]-9,9-spirobifluorene, 6-methyl-2-(4-(9-(4-(6-methylbenzo[d]thiazol-2-yl)phenyl)anthracen-10-yl)phenyl)benzo-[d]thiazole, 1,4-di[4-(N,N-diphenyl)amino]styrylbenzene, 1,4-bis(4-(9H-carbazol-9-yl)styryl)benzene, (E)-6-(4-(diphenylamino)styryl)-N,N-diphenylnaphthalen-2-amine, bis(2,4-difluorophenylpyridinato)(5-(pyridin-2-yl)-1H-tetrazolate) iridium(III), bis(3-trifluoromethyl-5-(2-pyridyl)pyrazole)((2,4-difluorobenzyl)diphenylphosphinate) iridium(III), bis(3-trifluoromethyl-5-(2-pyridyl)pyrazolate)(benzyldiphenylphosphinate) iridium(III), bis(1-(2,4-difluorobenzyl)-3-methylbenzimidazolium)(3-(trifluoromethyl)-5-(2-pyridyl)-1,2,4-triazolate) iridium(III), bis(3-trifluoromethyl-5-(2-pyridyl)pyrazolate)(4',6'-difluorophenylpyridinate) iridium(III), bis(4',6'-difluorophenylpyridinato)(3,5-bis(trifluoromethyl)-2-(2'-pyridyl)pyrrolate) iridium(III), bis(4',6'-difluorophenylpyridinato)(3-(trifluoromethyl)-5-(2-pyridyl)-1,2,4-triazolate) iridium (III), (Z)-6-mesityl-N-(6-mesitylquinolin-2(1H)-ylidene)quinoline-2-amine-BF₂, (E)-2-(2-(4-(dimethylamino)styryl)-6-methyl-4H-pyran-4-ylidene)malononitrile, 4-(dicyanomethylene)-2-methyl-6-julolidyl-9-enyl-4H-pyran, 4-(dicyanomethylene)-2-methyl-6-(1,1,7,7-tetramethyljulolidyl-9-enyl)-4H-pyran, 4-(dicyanomethylene)-2-tert-butyl-6-(1,1,7,7-tetramethyljulolidin-4-ylvinyl)-4H-pyran, tris(dibenzoylmethane)phenanthroline europium(III), 5,6,11,12-tetraphenylnaphthacene, bis(2-benzo[b]thiophen-2-yl-pyridine)(acetylacetonate) iridium(III), tris(1-phenylisoquinoline) iridium (III), bis(1-phenylisoquinoline)(acetylacetonate) iridium(III), bis[1-(9,9-dimethyl-9H-fluoren-2-yl)isoquinoline](acetylacetonate) iridium(III), bis[2-(9,9-dimethyl-9H-fluoren-2-yl)quinoline](acetylacetonate) iridium(III), tris[4,4' -di-tert-butyl-(2,2')-bipyridine] ruthenium(III) bis(hexafluorophosphate), tris(2-phenylquinoline) iridium(III), bis(2-phenylquinoline)(acetylacetonate) iridium(III), 2,8-di-tert-butyl-5,11-bis(4-tert-butylphenyl)-6,12-diphenyltetracene, bis(2-phenylbenzothiazolate)(acetylacetonate) iridium(III), platinum 5,10,15,20-tetraphenyltetrabenzoporphyrin, osmium(II) bis(3-trifluoromethyl-5-(2-pyridine)pyrazolate)dimethylphenylphosphine, osmium(II) bis(3-trifluoromethyl)-5-(4-tert-butylpyridyl)-1,2,4-triazolate)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolate)dimethylphenylphosphine, bis[2-(4-n-hexylphenyl)quinoline](acetylacetonate) iridium(III), tris[2-(4-n-hexylphenyl)quinoline] iridium(III), tris[2-phenyl-4-methylquinoline] iridium(III), bis(2-phenylquinoline)(2-(3-methylphenyl)pyridinate) iridium(III), bis(2-(9,9-diethylfluoren-2-yl)-1-phenyl-1H-benzo[d]imidazolato)(acetylacetonate) iridium(III), bis(2-phenylpyridine)(3-(pyridin-2-yl)-2H-chromen-2-onate) iridium(III), bis(2-phenylquinoline)(2,2,6,6-tetramethylheptane-3,5-dionate) iridium(III), bis(phenylisoquinoline)(2,2,6,6-tetramethylheptane-3,5-dionate) iridium(III), iridium(III) bis(4-phenylthieno[3,2-c]pyridinato-N,C²)acetylacetonate, (E)-2-(2-tert-butyl-6-(2-(2,6,6-trimethyl-2,4,5,6-tetrahydro-1H-pyrrolo[3,2,1-ij]quinolin-8-yl)vinyl)-4H-pyran-4-ylidene)malononitrile, bis(3-trifluoromethyl-5-(1-isoquinolyl)pyrazolate)(methyldiphenylphosphine) ruthenium, bis[(4-n-hexylphenyl)isoquinoline](acetylacetonate) iridium(III), platinum(II) octaethylporphin, bis(2-methyldibenzo[f,h]quinoxaline)(acetylacetonate) iridium(III) and tris[(4-n-hexylphenyl)isoquinoline] iridium(III).

Specific examples of electron-transporting layer-forming materials include lithium 8-hydroxyquinolinate, 2,2',2"-(1,3,5-benzinetriyl)-tris(1-phenyl-1-H-benzimidazole), 2-(4-biphenyl)-5-(4-tert-butylphenyl)-1,3,4-oxadiazole, 2,9-dimethyl-4,7-diphenyl-1,10-phenanthroline, 4,7-diphenyl-1,10-phenanthroline, bis(2-methyl-8-quinolinolate)-4-(phenylphenolato)aluminum, 1,3-bis[2-(2,2'-bipyridin-6-yl)-1,3,4-oxadiazo-5-yl]benzene, 6,6'-bis[5-(biphenyl-4-yl)-1,3,4-oxadiazo-2-yl]-2,2'-bipyridine, 3-(4-biphenyl)-4-phenyl-5-tert-butylphenyl-1,2,4-triazole, 4-(naphthalen-1-yl)-3,5-diphenyl-4H-1,2,4-triazole, 2,9-bis(naphthalen-2-yl)-4,7-diphenyl-1,10-phenanthroline, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, tris(2,4,6-trimethyl-3-(pyridin-3-yl)phenyl)borane, 1-methyl-2-(4-(naphthalen-2-yl)phenyl)-1H-imidazo[4,5f][1,10]phenanthroline, 2-(naphthalen-2-yl)-4,7-diphenyl-1, 10-phenanthroline, phenyldipyrenylphosphine oxide, 3,3',5,5'-tetra[(m-pyridyl)-phen-3-yl]biphenyl, 1,3,5-tris[(3-pyridyl)-phen-3-yl]benzene, 4,4'-bis(4,6-diphenyl-1,3,5-triazin-2-yl)biphenyl, 1,3-bis[3,5-di(pyridin-3-yl)phenyl]benzene, bis(10-hydroxybenzo[h]quinolinato)beryllium, diphenylbis(4-(pyridin-3-yl)phenyl)silane and 3,5-di(pyren-1-yl)pyridine.

Examples of electron-injecting layer-forming materials include lithium oxide (Li₂O), magnesium oxide (MgO), alumina (All₂O₃), lithium fluoride (LiF), sodium fluoride (NaF), magnesium fluoride (MgF₂), cesium fluoride (CsF), strontium fluoride (SrF₂), molybdenum trioxide (MoO₃), aluminum, lithium acetylacetonate (Li(acac)), lithium acetate and lithium benzoate.

Examples of cathode materials include aluminum, magnesium-silver alloys, aluminum-lithium alloys, lithium, sodium, potassium and cesium.

Another example is described below of a method for producing the organic EL device of the invention in which a thin-film obtained from the charge-transporting varnish of the invention serves as the hole-injecting layer.

An organic EL device having a charge-transporting thin film formed with the charge-transporting varnish of the invention can be produced by, in the organic EL device production method described above, successively forming a hole-transporting layer and a light-emitting layer instead of carrying out vacuum evaporation operations for a hole transporting layer, a light-emitting layer, an electron-transporting layer and an electron-injecting layer. Specifically, the charge-transporting varnish of the invention is applied onto an anode substrate, and a hole-injecting layer is formed by the above described method. A hole-transporting layer and a light-emitting layer are then successively formed thereon, following which a cathode material is vapor-deposited on top, thereby giving an organic EL device.

The cathode and anode materials used here may be similar to those described above, and similar cleaning treatment and surface treatment may be carried out.

The method of forming the hole-transporting layer and the light-emitting layer is exemplified by a film-forming method that involves adding a solvent to a hole-transporting polymer material or a light-emitting polymer material, or to the material obtained by adding a dopant to either of these, thereby dissolving or uniformly dispersing the material, and then applying the solution or dispersion onto the hole-injecting layer or the hole-transporting layer and subsequently firing.

Examples of hole-transporting polymer materials include poly[(9,9-dihexylfluorenyl-2,7-diyl)-co-(N,N'-bis{p-butylphenyl}-1,4-diaminophenylene)], poly[(9,9-dioctylfluorenyl-2,7-diyl)-co-(N,N'-bis{p-butylphenyl}-1,1'-biphenylene-4,4-diamine)], poly[(9,9-bis{1'-penten-5'-yl}fluorenyl-2,7-diyl)-co-(N,N'-bis{p-butylphenyl}-1,4-diaminophenylene)], poly[N,N'-bis(4-butylphenyl)-N,N'-bis(phenyl)-benzidine] end-capped with polysilsesquioxane and poly[(9,9-dioctylfluorenyl-2,7-diyl)-co-(4,4'-(N-(p-butylphenyl))diphenylamine)] (TFB).

Examples of light-emitting polymer materials include polyfluorene derivatives such as poly(9,9-dialkylfluorene) (PDAF), poly(phenylene vinylene) derivatives such as poly(2-methoxy-5-(2'-ethylhexoxy)-1,4-phenylene vinylene) (MEH-PPV), polythiophene derivatives such as poly(3-alkylthiophene) (PAT), and polyvinylcarbazole (PVCz).

Examples of solvents include toluene, xylene and chloroform. Examples of the method of dissolution or uniform dispersion include stirring, stirring under applied heat, and ultrasonic dispersion.

Examples of the method of application include, but are not particularly limited to, inkjet coating, spraying, dipping, spin coating, transfer printing, roll coating and brush coating. Application is preferably carried out in an inert gas atmosphere such as nitrogen or argon.

Examples of the firing method include methods that involve heating in an oven or on a hot plate, either within an inert gas atmosphere or in a vacuum.

An example is described below of a method for producing the organic EL device of the invention in cases where a thin film obtained from the charge-transporting varnish of the invention serves as a hole-injecting-and-transporting layer.

A hole-injecting-and-transporting layer is formed on an anode substrate. A light emitting layer, an electron-transporting layer, an electron-injecting layer and a cathode are provided in this order on the hole-injecting-and-transporting layer. Methods of forming the light-emitting layer, electron-transporting layer and electron-injecting layer, and specific examples thereof, include the same as those mentioned above.

The anode material, the light-emitting layer, the light-emitting dopant, the materials which form the electron-transporting layer and the electron-blocking layer, and the cathode material are exemplified in the same way as mentioned above.

A hole-blocking layer, an electron-blocking layer or the like may be optionally provided between the electrodes and any of the above layers. By way of illustration, an example of a material that forms an electron-blocking layer is tris(phenylpyrazole)iridium.

The materials which make up the anode, the cathode and the layers formed therebetween differ according to whether a device provided with a bottom emission structure or a top emission structure is to be fabricated, and so are suitably selected while taking this into account.

Typically, in a device having a bottom emission structure, a transparent anode is used on the substrate side and light is extracted from the substrate side, whereas in a device having a top emission structure, a reflective anode made of metal is used and light is extracted from the transparent electrode (cathode) side in the opposite direction from the substrate. Hence, for example, with regard to the anode material, when fabricating a device having a bottom emission structure, a transparent anode of ITO or the like is used, and when fabricating a device having a top emission structure, a reflective anode of Al/Nd or the like is used.

To prevent deterioration of the device characteristics, the organic EL device of the invention may be sealed in the usual manner with, if necessary, a desiccant or the like.

### EXAMPLES

Examples and Comparative Examples are given below to more concretely illustrate the invention, although the invention is not limited by these Examples. In the Examples, the following equipment was used for sample preparation and for analyzing physical properties.

| | |
|---|---|
| (1) ¹H-NMR Measurement: | Ascend 500, from Bruker |
| (2) LC/MS: | ZQ 2000, from Waters Corporation |
| (3) Substrate Cleaning: | Substrate cleaning machine (reduced-pressure plasma system), from Choshu Industry Co., Ltd. |
| (4) Varnish Coating: | MS-A100 Spin Coater, from Mikasa Co., Ltd. |
| (5) Film Thickness Measurement: | Surfcorder ET-4000 microfigure measuring instrument, from Kosaka Laboratory, Ltd. |
| (6) HOD Fabrication: | C-E2L1G1-N Multifunction Vapor Deposition System, from Choshu Industry Co., Ltd. |
| (7) HOD Current-Voltage Measurement: | I-V-L Measurement System, from EHC Co., Ltd. |

### [1] Synthesis of Compounds

### [Comparative Example 1-1] Synthesis of Sulfonic Acid Compound 4FNS-4

The sulfonic acid compound 4FNS-4 of the following formula was synthesized in accordance with the method described in WO 2015/111654.

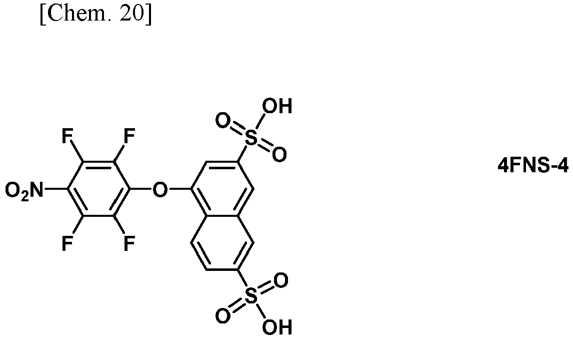

### [Comparative Example 1-2] Synthesis of Sulfonic Acid Compound 4FNS-2

The sulfonic acid compound 4FNS-2 of the following formula was synthesized in accordance with the method described in WO 2009/096352.

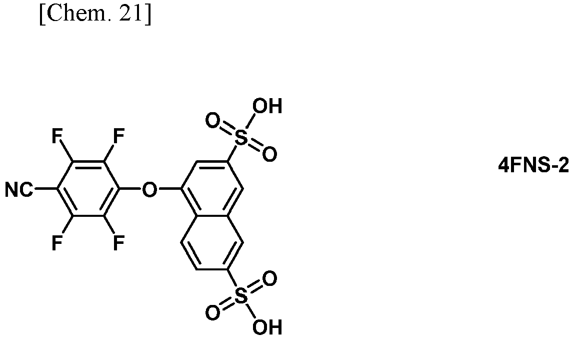

### [Example 1-1] Synthesis of Sulfonic Acid Ester Compound 4FNS-4-PGEE

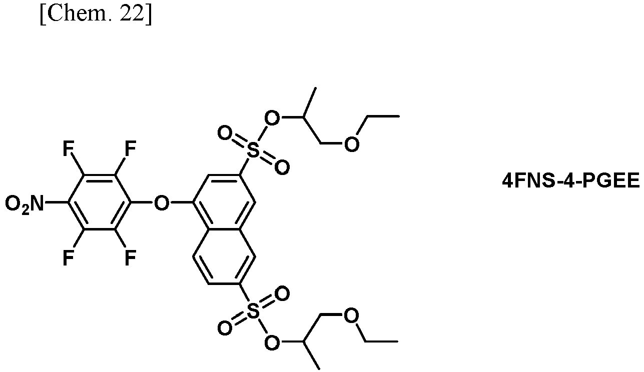

Thionyl chloride (25 g) and N,N-dimethylformamide (0.4 mL) as a catalyst were added to 4FNS-4 (4.97 g, 10 mmol), and the system was refluxed under heating for 1 hour, following which the thionyl chloride was driven off, giving a solid containing an acid chloride of 4FNS-4. This compound was used in the next step without further purification.

Chloroform (30 mL) and pyridine (20 mL) were added to the solid, and 6.24 g (60 mmol) of propylene glycol monoethyl ether was added at 0°C. The temperature was raised to room temperature and 1.5 hours of stirring was carried out thereafter. The solvent was driven off, following which water was added, extraction was carried out with ethyl acetate, and the organic layer was dried over sodium sulfate. After filtration and concentration, the resulting crude product was purified by silica gel column chromatography (hexane/ethyl acetate), giving 1.32 g of the sulfonic acid ester compound 4FNS-4-PGEE as a white solid (yield: 20% (2-step yield from 4FNS-4)). The results of ¹H-NMR and LC/MS measurement are shown below.
¹H-NMR (500 MHz, CDCl₃):
δ 0.89-0.95 (m, 6H), 1.34 and 1.39 (a pair of d, J = 6.5 Hz, 6H), 3.28-3.50 (m, 8H), 4.81-4.87 (m, 2H), 7.26 (s, 1H), 8.22 (d, J = 9.0 Hz, 1H), 8.47 (s, 1H), 8.54 (d, J = 9.0 Hz, 1H), 8.68 (s, 1H).
LC/MS (ESI⁺) m/z; 687 [M+NH₄]⁺

### [Example 1-2] Synthesis of Sulfonic Acid Ester Compound 4FNS-2-PGEE

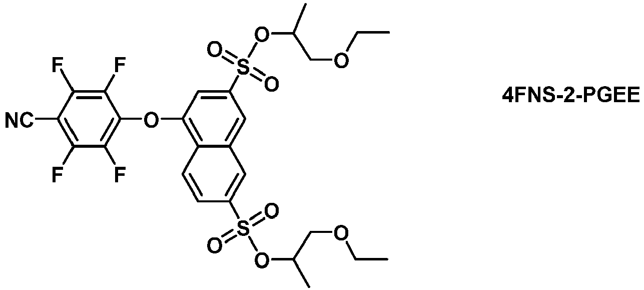

Except that 4FNS-2 (4.77g, 10 mmol) was used instead of 4FNS-4, synthesis was carried out in the same manner as in Example 1-1 to give 1.04 g of sulfonic acid ester compound 4FNS-2-PGEE as a white solid (yield: 16% (2-step yield from 4FNS-2)). The results of ¹H-NMR and LC/MS measurement are shown below.
¹H-NMR (500 MHz, CDCl₃):
δ 0.89-0.95 (m, 6H), 1.33 and 1.39 (a pair of d, J = 6.5 Hz, 6H), 3.28-3.50 (m, 8H), 4.77-4.89 (m, 2H), 7.25 (s, 1H), 8.22 (d, J = 8.8 Hz, 1H), 8.45 (s, 1H), 8.52 (d, J = 8.8 Hz, 1H), 8.68 (s, 1H).
LC/MS (ESI⁺) m/z; 667 [M+NH₄]⁺

### [Example 1-3] Synthesis of Sulfonic Acid Ester Compound 4FNS-4-EH

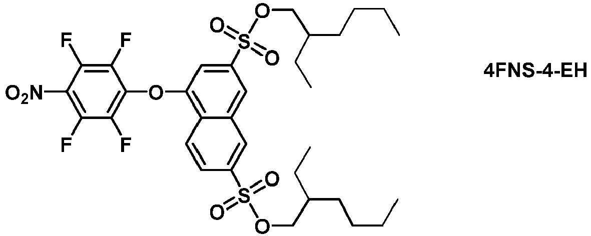

Except that 2-ethyl-1-hexanol was used instead of propylene glycol monoethyl ether, synthesis was carried out in the same manner as in Example 1-1 to give 1.48 g of sulfonic acid ester compound 4FNS-4-EH as a white solid from 4FNS-4 (4.97 g, 10 mmol) (yield: 21% (2-step yield from 4FNS-4)). The measurement results of ¹H-NMR are shown below.
¹H-NMR (500 MHz, CDCl₃):
δ 0.77-0.84 (m, 12H), 1.13-1.39 (m, 16H), 1.54-1.62 (m, 2H), 3.99-4.05 (m, 2H), 4.05-4.11 (m, 2H), 7.24 (s, 1H), 8.20 (d, J = 8.9 Hz, 1H), 8.45 (s, 1H), 8.60 (d, J = 8.9 Hz, 1H), 8.69 (s, 1H).

### [2] Preparation of Charge-Transporting Varnishes and Evaluation of Solubility

### [Example 2-1] Preparation of Charge-Transporting Varnish A1

The 4FNS-4-PGEE (348 mg) and Oligoaniline Compound 1 (178 mg) were added to a mixed solvent of 3-phenoxytoluene (3 g; dielectric constant: 2.7) and butyl benzoate (7 g; dielectric constant: 2.5), and the system was stirred for 10 minutes under heating at 50°C and 350 rpm. As a result, the 4FNS-4-PGEE was dissolved completely in the solvent. The resulting solution was filtered using a PTFE filter having a pore size of 0.2 µm, giving charge-transporting varnish A1 (solid concentration: 5% by weight). Oligoaniline Compound 1 was synthesized in accordance with the method described in WO 2013/084664.

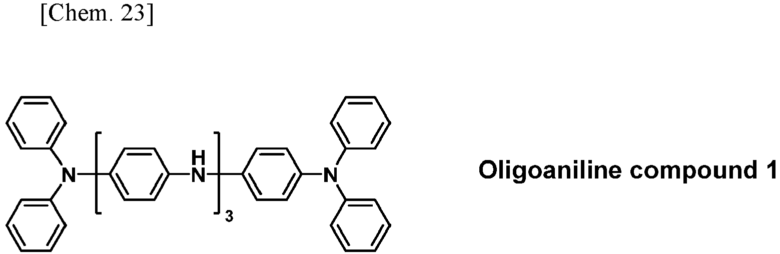

### [Example 2-2] Preparation of Charge-Transporting Varnish A2

The 4FNS-4-PGEE (257 mg) and Oligoaniline Compound 2 (270 mg) were added to a mixed solvent of 3-phenoxytoluene (3 g) and butyl benzoate (7 g), and the system was stirred for 10 minutes under heating at 50°C and 350 rpm. As a result, the 4FNS-4-PGEE was dissolved completely in the solvent. The resulting solution was filtered using a PTFE filter having a pore size of 0.2 µm, giving charge-transporting varnish A2 (solid concentration: 5% by weight). Oligoaniline Compound 2 was synthesized in accordance with the method described in WO 2015/050253, Synthesis Example 18.

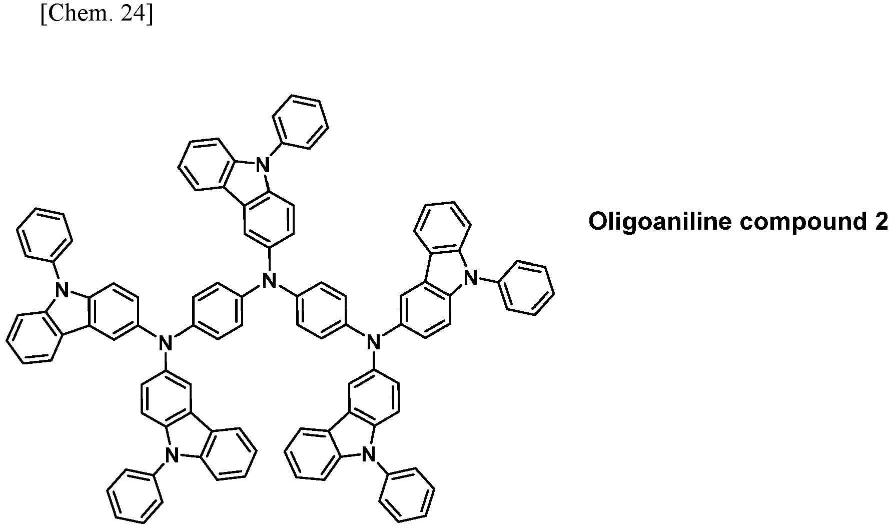

### [Example 2-3] Preparation of Charge-Transporting Varnish A3

The 4FNS-2-PGEE (253 mg) and Oligoaniline Compound 2 (274 mg) were added to a mixed solvent of 3-phenoxytoluene (3 g) and butyl benzoate (7 g), and the system was stirred for 10 minutes under heating at 50°C and 350 rpm. As a result, the 4FNS-2-PGEE was dissolved completely in the solvent. The resulting solution was filtered using a PTFE filter having a pore size of 0.2 µm, giving charge-transporting varnish A3 (solid concentration: 5% by weight).

### [Example 2-4] Preparation of Charge-Transporting Varnish A4

The 4FNS-4-EH (267 mg) and Oligoaniline Compound 2 (260 mg) were added to a mixed solvent of 3-phenoxytoluene (3 g) and butyl benzoate (7 g), and the system was stirred for 10 minutes under heating at 50°C and 350 rpm. As a result, the 4FNS-4-EH was dissolved completely in the solvent. The resulting solution was filtered using a PTFE filter having a pore size of 0.2 µm, giving charge-transporting varnish A4 (solid concentration: 5% by weight).

### [Example 2-5] Preparation of Charge-Transporting Varnish A5

The 4FNS-4-EH (357 mg) and Oligoaniline Compound 1 (170 mg) were added to a mixed solvent of 3-phenoxytoluene (3 g) and butyl benzoate (7 g), and the system was stirred for 10 minutes under heating at 50°C and 350 rpm. As a result, the 4FNS-4-EH was dissolved completely in the solvent. The resulting solution was filtered using a PTFE filter having a pore size of 0.2 µm, giving charge-transporting varnish A5 (solid concentration: 5% by weight).

### [Example 2-6] Preparation of Charge-Transporting Varnish A6

The 4FNS-2-PGEE (345 mg) and Oligoaniline Compound 1 (182 mg) were added to a mixed solvent of 3-phenoxytoluene (3 g) and butyl benzoate (7 g), and the system was stirred for 10 minutes under heating at 50°C and 350 rpm. As a result, the 4FNS-2-PGEE was dissolved completely in the solvent. The resulting solution was filtered using a PTFE filter having a pore size of 0.2 µm, giving charge-transporting varnish A6 (solid concentration: 5% by weight).

### [Example 2-7] Preparation of Charge-Transporting Varnish B1

The 4FNS-4-PGEE (135 mg) and Oligoaniline Compound 1 (69 mg) were added to a mixed solvent of 1,3-dimethyl-2-imidazolidinone (3.3 g; dielectric constant: 26.0), 2,3-butanediol (4 g; dielectric constant: 17.0) and diethylene glycol monoethyl ether (2.7 g; dielectric constant: 7.9), and the system was stirred for 10 minutes under heating at 50°C and 350 rpm. As a result, the 4FNS-4-PGEE was dissolved completely in the solvent. The resulting solution was filtered using a PTFE filter having a pore size of 0.2 µm, giving charge-transporting varnish B1 (solid concentration: 2% by weight).

### [Example 2-8] Preparation of Charge-Transporting Varnish B2

The 4FNS-4-PGEE (100 mg) and Oligoaniline Compound 2 (105 mg) were added to a mixed solvent of diethylene glycol (4 g; dielectric constant: 25.2) and triethylene glycol dimethyl ether (6 g; dielectric constant: 5.1), and the system was stirred for 10 minutes under heating at 50°C and 350 rpm. As a result, the 4FNS-4-PGEE was dissolved completely in the solvent. The resulting solution was filtered using a PTFE filter having a pore size of 0.2 µm, giving charge-transporting varnish B2 (solid concentration: 2% by weight).

### [Example 2-9] Preparation of Charge-Transporting Varnish B3

The 4FNS-2-PGEE (98 mg) and Oligoaniline Compound 2 (106 mg) were added to a mixed solvent of diethylene glycol (4 g) and triethylene glycol dimethyl ether (6 g), and the system was stirred for 10 minutes under heating at 50°C and 350 rpm. As a result, the 4FNS-2-PGEE was dissolved completely in the solvent. The resulting solution was filtered using a PTFE filter having a pore size of 0.2 µm, giving charge-transporting varnish B3 (solid concentration: 2% by weight).

### [Example 2-10] Preparation of Charge-Transporting Varnish B4

The 4FNS-4-EH (103 mg) and Oligoaniline Compound 2 (101 mg) were added to a mixed solvent of diethylene glycol (4 g) and triethylene glycol dimethyl ether (6 g), and the system was stirred for 10 minutes under heating at 50°C and 350 rpm. As a result, the 4FNS-4-EH was dissolved completely in the solvent. The resulting solution was filtered using a PTFE filter having a pore size of 0.2 µm, giving charge-transporting varnish B4 (solid concentration: 2% by weight).

### [Example 2-11] Preparation of Charge-Transporting Varnish B5

The 4FNS-4-EH (138 mg) and Oligoaniline Compound 1 (66 mg) were added to a mixed solvent of 1,3-dimethyl-2-imidazolidinone (3.3 g), 2,3-butanediol (4 g) and diethylene glycol monoethyl ether (2.7 g), and the system was stirred for 10 minutes under heating at 50°C and 350 rpm. As a result, the 4FNS-4-EH was dissolved completely in the solvent. The resulting solution was filtered using a PTFE filter having a pore size of 0.2 µm, giving charge-transporting varnish B5 (solid concentration: 2% by weight).

### [Comparative Example 2-1]

The 4FNS-4 (312 mg) and Oligoaniline Compound 1 (215 mg) were added to a mixed solvent of 3-phenoxytoluene (3 g) and butyl benzoate (7 g), and the system was stirred for 60 minutes under heating at 50°C and 350 rpm. The 4FNS-4 was not dissolved.

### [Comparative Example 2-2]

The 4FNS-4 (218 mg) and Oligoaniline Compound 2 (308 mg) were added to a mixed solvent of 3-phenoxytoluene (3 g) and butyl benzoate (7 g), and the system was stirred for 60 minutes under heating at 50°C and 350 rpm. The 4FNS-4 was not dissolved.

### [Comparative Example 2-3]

The 4FNS-2 (306 mg) and Oligoaniline Compound 1 (220 mg) were added to a mixed solvent of 3-phenoxytoluene (3 g) and butyl benzoate (7 g), and the system was stirred for 60 minutes under heating at 50°C and 350 rpm. The 4FNS-2 was not dissolved.

### [Comparative Example 2-4]

The 4FNS-2 (213 mg) and Oligoaniline Compound 2 (313 mg) were added to a mixed solvent of 3-phenoxytoluene (3 g) and butyl benzoate (7 g), and the system was stirred for 60 minutes under heating at 50°C and 350 rpm. The 4FNS-2 was not dissolved.

### [Comparative Example 2-5] Preparation of Charge-Transporting Varnish C1

The 4FNS-4 (121 mg) and Oligoaniline Compound 1 (83 mg) were added to a mixed solvent of 1,3-dimethyl-2-imidazolidinone (3.3 g), 2,3-butanediol (4 g) and diethylene glycol monoethyl ether (2.7 g), and the system was stirred for 10 minutes under heating at 50°C and 350 rpm. As a result, the 4FNS-4 was dissolved completely in the solvent. The resulting solution was filtered using a PTFE filter having a pore size of 0.2 µm, giving charge-transporting varnish C1 (solid concentration: 2% by weight).

### [Comparative Example 2-6] Preparation of Charge-Transporting Varnish C2

The 4FNS-4 (85 mg) and Oligoaniline Compound 2 (120 mg) were added to a mixed solvent of diethylene glycol (4 g) and triethylene glycol dimethyl ether (6 g), and the system was stirred for 10 minutes under heating at 50°C and 350 rpm. As a result, the 4FNS-4 was dissolved completely in the solvent. The resulting solution was filtered using a PTFE filter having a pore size of 0.2 µm, giving charge-transporting varnish C2 (solid concentration: 2% by weight).

### [Comparative Example 2-7] Preparation of Charge-Transporting Varnish C3

The 4FNS-2 (119 mg) and Oligoaniline Compound 1 (85 mg) were added to a mixed solvent of 1,3-dimethyl-2-imidazolidinone (3.3 g), 2,3-butanediol (4 g) and diethylene glycol monoethyl ether (2.7 g), and the system was stirred for 10 minutes under heating at 50°C and 350 rpm. As a result, the 4FNS-2 was dissolved completely in the solvent. The resulting solution was filtered using a PTFE filter having a pore size of 0.2 µm, giving charge-transporting varnish C3 (solid concentration: 2% by weight).

### [Comparative Example 2-8] Preparation of Charge-Transporting Varnish C4

The 4FNS-2 (83 mg) and Oligoaniline Compound 2 (122 mg) were added to a mixed solvent of diethylene glycol (4 g) and triethylene glycol dimethyl ether (6 g), and the system was stirred for 10 minutes under heating at 50°C and 350 rpm. As a result, the 4FNS-2 was dissolved completely in the solvent. The resulting solution was filtered using a PTFE filter having a pore size of 0.2 µm, giving charge-transporting varnish C4 (solid concentration: 2% by weight).

4FNS-4-PGEF, 4FNS-2-PGEE and 4FNS-4-EH were dissolved completely in a mixed solvent of the low-polarity solvents 3-phenoxytoluene and butyl benzoate when stirred for 10 minutes under heating at 50°C and 350 rpm, whereas 4FNS-4 and 4FNS-2 were not dissolved in the mixed solvent. 4FNS-4-PGEE, 4FNS-2-PGEE, 4FNS-4-EH, 4FNS-4, and 4FNS-2 were all dissolved completely in the high-polarity solvent. The results showed that when esterified as in the invention, the sulfonic acid compound became soluble in both the low-polarity solvent and the high-polarity solvent, leading to expansion of solvent selectivity.

### [3] Fabrication of Top Layer Deposition-Type Hole-Only Devices (HOD) and Evaluation of Device Characteristics

In the following Working Examples and Comparative Examples, a glass substrate with dimensions of 25 mm × 25 mm × 0.7 t and having ITO patterned on the surface to a film thickness of 150 nm was used as the ITO substrate. Prior to use, impurities on the surface were removed with an O₂ plasma cleaning system (150 W, 30 seconds).

### [Example 3-1]

Charge-transporting varnish A1 was applied onto the ITO substrate using a spin coater and was subsequently pre-fired at 120°C for 1 minute in open air and then subjected to main firing at 230°C for 15 minutes, thereby forming a 40-nm thin film on the ITO substrate.

Using a vapor deposition system (degree of vacuum: 2.0 × 10⁻⁵ Pa), thin films of α-NPD and aluminum were successively deposited thereon, giving a hole-only device. Vapor deposition was carried out at a deposition rate of 0.2 nm/s. The thicknesses of the α-NPD thin film and the aluminum thin film were set to respectively 30 nm and 80 nm.

To prevent the device characteristics from deteriorating due to the influence of oxygen, moisture and the like in air, the hole-only device was sealed with sealing substrates, following which the characteristics were evaluated. Sealing was carried out by the following procedure.

The hole-only device was placed between sealing substrates in a nitrogen atmosphere having an oxygen concentration of 2 ppm or less and a dew point of not more than -85°C, and the sealing substrates were laminated together using an adhesive (MORESCO Moisture Cut WB90US(P), from Moresco Corporation). At this time, a desiccant (HD-071010W-40, from Dynic Corporation) was placed, together with the hole-only device, within the sealing substrates. The laminated sealing substrates were irradiated with UV light (wavelength, 365 nm; dosage, 6,000 mJ/cm²) and then annealed at 80°C for 1 hour to cure the adhesive.

### [Example 3-2]

Except that charge-transporting varnish A2 was used instead of charge-transporting varnish A1, the same procedure as in Example 3-1 was carried out to fabricate a hole-only device.

### [Example 3-3]

Except that charge-transporting varnish A3 was used instead of charge-transporting varnish A1, the same procedure as in Example 3-1 was carried out to fabricate a hole-only device.

### [Example 3-4]

Except that charge-transporting varnish A4 was used instead of charge-transporting varnish A1, the same procedure as in Example 3-1 was carried out to fabricate a hole-only device.

### [Example 3-5]

Except that charge-transporting varnish A5 was used instead of charge-transporting varnish A1, the same procedure as in Example 3-1 was carried out to fabricate a hole-only device.

### [Example 3-6]

Except that charge-transporting varnish A6 was used instead of charge-transporting varnish A1, the same procedure as in Example 3-1 was carried out to fabricate a hole-only device.

### [Example 3-7]

Except that charge-transporting varnish B1 was used instead of charge-transporting varnish A1, the same procedure as in Example 3-1 was carried out to fabricate a hole-only device.

### [Example 3-8]

Except that charge-transporting varnish B2 was used instead of charge-transporting varnish A1, the same procedure as in Example 3-1 was carried out to fabricate a hole-only device.

### [Example 3-9]

Except that charge-transporting varnish B3 was used instead of charge-transporting varnish A1, the same procedure as in Example 3-1 was carried out to fabricate a hole-only device.

### [Example 3-10]

Except that charge-transporting varnish B4 was used instead of charge-transporting varnish A1, the same procedure as in Example 3-1 was carried out to fabricate a hole-only device.

### [Example 3-11]

Except that charge-transporting varnish B5 was used instead of charge-transporting varnish A1, the same procedure as in Example 3-1 was carried out to fabricate a hole-only device.

### [Comparative Example 3-1]

Except that charge-transporting varnish C1 was used instead of charge-transporting varnish A1, the same procedure as in Example 3-1 was carried out to fabricate a hole-only device.

### [Comparative Example 3-2]

Except that charge-transporting varnish C2 was used instead of charge-transporting varnish A1, the same procedure as in Example 3-1 was carried out to fabricate a hole-only device.

### [Comparative Example 3-3]

Except that charge-transporting varnish C3 was used instead of charge-transporting varnish A1, the same procedure as in Example 3-1 was carried out to fabricate a hole-only device.

### [Comparative Example 3-4]

Except that charge-transporting varnish C4 was used instead of charge-transporting varnish A1, the same procedure as in Example 3-1 was carried out to fabricate a hole-only device.

The current densities at a driving voltage of 4 V were measured for the hole-only devices fabricated in Examples 3-1 to 3-11 and Comparative Examples 3-1 to 3-4. The results are shown in Tables 1 to 4.

**[Table 1]**

| | Charge-transportability Varnish | Host | Dopant | Current density (mA/cm²) |
|---|---|---|---|---|
| Example 3-1 | A1 | Oligoaniline Compound 1 | 4FNS-4-PGEE | 1,999 |
| Example 3-7 | B1 | Oligoaniline Compound 1 | 4FNS-4-PGEE | 2,039 |
| Example 3-5 | A5 | Oligoaniline Compound 1 | 4FNS-4-EH | 1,970 |
| Example 3-11 | B5 | Oligoaniline Compound 1 | 4FNS-4-EH | 1,490 |
| Comparative Example 3-1 | C1 | Oligoaniline Compound 1 | 4FNS-4 | 722 |

**[Table 2]**

| | Charge-transportability Varnish | Host | Dopant | Current density (mA/cm²) |
|---|---|---|---|---|
| Example 3-2 | A2 | Oligoaniline Compound 2 | 4FNS-4-PGEE | 2,370 |
| Example 3-8 | B2 | Oligoaniline Compound 2 | 4FNS-4-PGEE | 2,086 |
| Example 3-4 | A4 | Oligoaniline Compound 2 | 4FNS-4-EH | 1,680 |
| Example 3-10 | B4 | Oligoaniline Compound 2 | 4FNS-4-EH | 2,110 |
| Comparative Example 3-2 | C2 | Oligoaniline Compound 2 | 4FNS-4 | 97 |

**[Table 3]**

| | Charge-transportability Varnish | Host | Dopant | Current density (mA/cm²) |
|---|---|---|---|---|
| Example 3-6 | A6 | Oligoaniline Compound 1 | 4FNS-2-PGEE | 433 |
| Comparative Example 3-3 | C3 | Oligoaniline Compound 1 | 4FNS-2 | 157 |

**[Table 4]**

| | Charge-transportability Varnish | Hos t | Dopant | Current density (mA/cm²) |
|---|---|---|---|---|
| Example 3-3 | A3 | Oligoaniline Compound 2 | 4FNS-2-PGEE | 1,250 |
| Example 3-9 | B3 | Oligoaniline Compound 2 | 4FNS-2-PGEE | 2,070 |
| Comparative Example 3-4 | C4 | Oligoaniline Compound 2 | 4FNS-2 | 1,141 |

As shown in Tables 1 to 4, a charge-transporting varnish containing the sulfonic acid ester compound of the invention had higher hole-transportability as compared to a charge-transporting varnish containing a conventional sulfonic acid ester compound.

### [4] Preparation of Charge-Transporting Varnishes and Evaluation of Storage Stability

### [Example 4-1] Preparation of Charge-Transporting Varnish D

The 4FNS-4-PGEE (348 mg) and Oligoaniline Compound 1 (178 mg) were added to a mixed solvent of 3-phenoxytoluene (5 g; dielectric constant: 2.7) and tetralin (5 g; dielectric constant: 2.2), and the system was stirred for 10 minutes under heating at 50°C and 350 rpm. As a result, the 4FNS-4-PGEE was dissolved completely in the solvent. The resulting solution was filtered using a PTFE filter having a pore size of 0.2 µm, giving charge-transporting varnish D (solid concentration: 5% by weight).

### [Comparative Example 4-1] Preparation of Charge-Transporting Varnish E

The NSO-2-PGME (384 mg) and Oligoaniline Compound 1 (142 mg) were added to a mixed solvent of 3-phenoxytoluene (5 g) and tetralin (5 g), and the system was stirred for 30 minutes under heating at 50°C and 400 rpm. Undissolved residues existed. Stirring was performed under heating at 70°C and 400 rpm for 20 minutes to dissolve the NSO-2-PGME completely in the solvent. The resulting solution was filtered using a PTFE filter having a pore size of 0.2 µm, giving charge-transporting varnish E (solid concentration: 5% by weight). The NSO-2-PGME was synthesized in accordance with the method described in Patent Document 6.

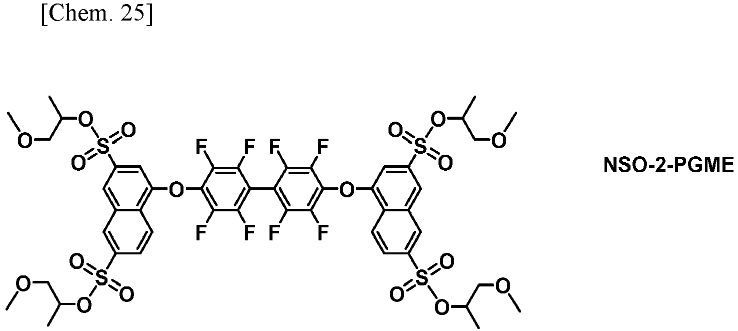

Charge-transporting varnishes D and E were stored under refrigeration at 2°C, and were visually examined for the existence or non-existence of precipitate when a period of 7 days had elapsed following the start of storage. The results are shown in Table 5.

**[Table 5]**

| | Charge-transporting varnish | Sulfonic acid ester compound | Existence or non-existence of precipitate |
|---|---|---|---|
| Example 4-1 | D | 4FNS-4-PGEE | None |
| Comparative Example 4-1 | E | NSO-2-PGME | Present |

As shown in Table 5, a charge-transporting varnish containing the sulfonic acid ester compound of the invention was excellent in storage stability.

## Claims

1. A Sulfonic acid ester compound of the following formula (1): 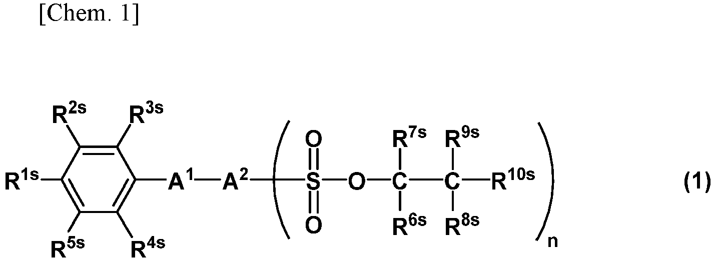
wherein R^{1s} to R^{5s} are each independently a hydrogen atom, a nitro group, a cyano group, a halogen atom, an alkyl group of 1 to 10 carbon atoms, a halogenated alkyl group of 1 to 10 carbon atoms, or a halogenated alkenyl group of 2 to 10 carbon atoms;
R^{6s} to R^{9s} are each independently a hydrogen atom, or a linear or branched monovalent aliphatic hydrocarbon group of 1 to 20 carbon atoms;
R^{10s} is a linear or branched monovalent aliphatic hydrocarbon group of 1 to 20 carbon atoms, or -OR^{11s}, where R^{11s} is an optionally substituted monovalent hydrocarbon group of 2 to 20 carbon atoms;
A¹ is -O-, -S- or -NH-;
A² is an (n+1)-valent aromatic group; and
n is an integer that satisfies the condition 1 ≤ n ≤ 4.

2. The sulfonic acid ester compound according to claim 1, wherein R^{1s} is a nitro group, a cyano group, a halogenated alkyl group of 1 to 10 carbon atoms, or a halogenated alkenyl group of 2 to 10 carbon atoms.

3. The sulfonic acid ester compound according to claim 1 or 2, wherein all of R^{2s} to R^{5s} are fluorine atoms.

4. The sulfonic acid ester compound according to any one of claims 1 to 3, wherein A² is a group derived from naphthalene.

5. The sulfonic acid ester compound according to any one of claims 1 to 4, wherein n is 2.

6. The sulfonic acid ester compound according to any one of claims 1 to 5, which is represented by the following formula (1-1): 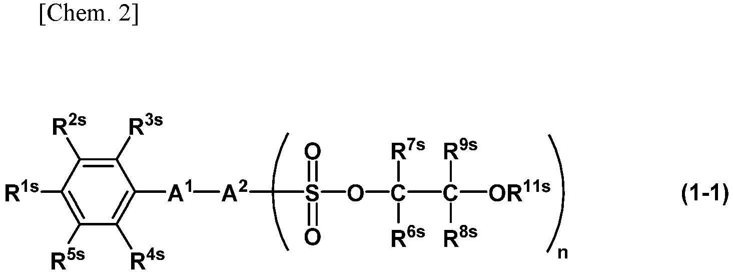 wherein R^{1s} to R^{9s}, R^{11s}, A¹, A² and n are the same as described above.

7. The sulfonic acid ester compound according to any one of claims 1 to 5, which is represented by the following formula (1-2): 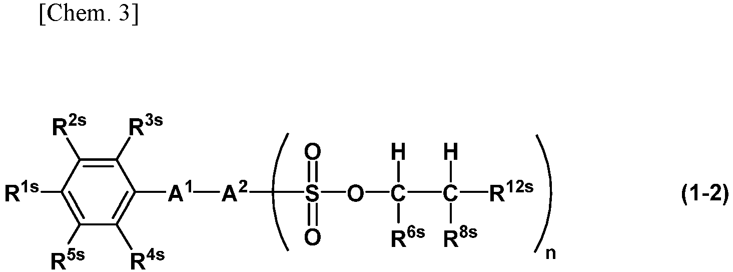 wherein R^{1s} to R^{6s}, R^{8s}, A¹, A² and n are the same as described above, and R^{12s} is a linear or branched monovalent aliphatic hydrocarbon group of 1 to 20 carbon atoms.

8. An electron-accepting substance precursor consisting of the sulfonic acid ester compound according to any one of claims 1 to 7.

9. A charge-transporting varnish comprising the electron-accepting substance precursor according to claim 8, a charge-transporting substance, and an organic solvent.

10. The charge-transporting varnish according to claim 9, wherein the organic solvent is a low-polarity organic solvent.

11. The charge-transporting varnish according to claim 9 or 10 above, wherein the charge-transporting substance is an aniline derivative.

12. A charge-transporting thin film obtained using the charge-transporting varnish according to any one of claims 9 to 11.

13. An organic electroluminescence device comprising the charge-transporting thin film according to claim 12.
